# EUROPEAN PATENT SPECIFICATION

(11) **EP 3 897 815 B1**
(45) Date of publication and mention of the grant of the patent: **30.10.2024**
(21) Application number: 19828092.7
(22) Date of filing: 04.12.2019
(51) Int. Cl.: A61N 1/05, A61N 1/365, A61N 1/372, A61N 1/375, A61B 6/12, A61N 1/368

(54) **IMPLANTABLE MEDICAL DEVICE DELIVERY FOR CARDIAC THERAPY**
IMPLANTIERBARE MEDIZINISCHE VORRICHTUNGSEINFÜHRUNG FÜR DIE HERZTHERAPIE
POSE DE DISPOSITIF MÉDICAL IMPLANTABLE POUR THÉRAPIE CARDIAQUE

(30) Priority: 20.12.2018 US 201816227774
(43) Date of publication of application: 27.10.2021
(73) Proprietor: Medtronic, Inc., Minneapolis, Minnesota 55432 (US)
(72) Inventor: DRAKE, Ronald A., Minneapolis, MN 55432 (US)
(74) Representative: Zimmermann & Partner Patentanwälte mbB
(86) International application number: PCT/US2019/064480
(87) International publication number: WO 2020/131384

(56) References cited:
- US-A1- 2013 116 738
- US-A1- 2016 242 887
- US-A1- 2018 256 902
- US-B1- 8 142 363
- US-B2- 10 004 467
- US-B2- 7 937 161

## Description

The present technology is generally related to implantable medical devices and systems. In particular, the present disclosure relates to delivering implantable medical devices to provide cardiac therapy.

The cardiac conduction system includes the sinus atrial (SA) node, the atrioventricular (AV) node, the bundle of His, bundle branches and Purkinje fibers. A heart beat is initiated in the SA node, which may be described as the natural "pacemaker" of the heart. An electrical impulse arising from the SA node causes the atrial myocardium to contract. The signal is conducted to the ventricles via the AV node which inherently delays the conduction to allow the atria to stop contracting before the ventricles begin contracting thereby providing proper AV synchrony. The electrical impulse is conducted from the AV node to the ventricular myocardium via the bundle of His, bundle branches, and Purkinje fibers.

Patients with a conduction system abnormality, such as poor AV node conduction or poor SA node function, may receive an implantable medical device (IMD), such as a pacemaker, to restore a more normal heart rhythm and AV synchrony. Some types of IMDs, such as cardiac pacemakers, implantable cardioverter defibrillators (ICDs), or cardiac resynchronization therapy (CRT) devices, provide therapeutic electrical stimulation to a heart of a patient via electrodes on one or more implantable endocardial, epicardial, or coronary venous leads that are positioned in or adjacent to the heart. The therapeutic electrical stimulation may be delivered to the heart in the form of pulses or shocks for pacing, cardioversion, or defibrillation. In some cases, an IMD may sense intrinsic depolarizations of the heart, and control the delivery of therapeutic stimulation to the heart based on the sensing.

Delivery of therapeutic electrical stimulation to the heart can be useful in addressing cardiac conditions such as ventricular dyssynchrony that may occur in patients. Ventricular dyssynchrony may be described as a lack of synchrony or a difference in the timing of contractions in different ventricles of the heart. Significant differences in timing of contractions can reduce cardiac efficiency. CRT, delivered by an IMD to the heart, may enhance cardiac output by resynchronizing the electromechanical activity of the ventricles of the heart. CRT is sometimes referred to as "triple chamber pacing" because of pacing the right atrium, right ventricle, and left ventricle.

Cardiac arrhythmias may be treated by delivering electrical shock therapy for cardioverting or defibrillating the heart in addition to cardiac pacing, for example, from an ICD, which may sense a patient's heart rhythm and classify the rhythm according to an arrhythmia detection scheme in order to detect episodes of tachycardia or fibrillation. Arrhythmias detected may include ventricular tachycardia (VT), fast ventricular tachycardia (FVT), ventricular fibrillation (VF), atrial tachycardia (AT) and atrial fibrillation (AT). Anti-tachycardia pacing (ATP), a painless therapy, can be used to treat ventricular tachycardia (VT) to substantially terminate many monomorphic fast rhythms. While ATP is painless, ATP may not deliver effective therapy for all types of VTs. For example, ATP may not be as effective for polymorphic VTs, which has variable morphologies. Polymorphic VTs and ventricular fibrillation (VFs) can be more lethal and may require expeditious treatment by shock.

Dual chamber medical devices are available that include a transvenous atrial lead carrying electrodes that may be placed in the right atrium and a transvenous ventricular lead carrying electrodes that may be placed in the right ventricle via the right atrium. The dual chamber medical device itself is generally implanted in a subcutaneous pocket and the transvenous leads are tunneled to the subcutaneous pocket. A dual chamber medical device may sense atrial electrical signals and ventricular electrical signals and can provide both atrial pacing and ventricular pacing as needed to promote a normal heart rhythm and AV synchrony. Some dual chamber medical devices can treat both atrial and ventricular arrhythmias.
US 7,937,161 B2 relates to cardiac stimulation electrodes, delivery devices, and implantation configurations.

Intracardiac medical devices, such as a leadless pacemaker, have been introduced or proposed for implantation entirely within a patient's heart, eliminating the need for transvenous leads. A leadless pacemaker may include one or more electrodes on its outer housing to deliver therapeutic electrical signals and/or sense intrinsic depolarizations of the heart Intracardiac medical devices may provide cardiac therapy functionality, such as sensing and pacing, within a single chamber of the patient's heart. Single chamber intracardiac devices may also treat either atrial or ventricular arrhythmias or fibrillation. Some leadless pacemakers are not intracardiac and may be positioned outside of the heart and, in some examples, may be anchored to a wall of the heart via a fixation mechanism.

In some patients, single chamber devices may adequately address the patient's needs. However, single chamber devices capable of only single chamber sensing and therapy may not fully address cardiac conduction disease or abnormalities in all patients, for example, those with some forms of AV dyssynchrony or tachycardia. Dual chamber sensing and/or pacing functions, in addition to ICD functionality in some cases, may be used to restore more normal heart rhythms.

### SUMMARY

The invention is defined by the independent claim 1. The techniques of this disclosure generally relate to delivering implantable medical devices to provide cardiac therapy using the cardiac conduction system or left ventricular myocardium implantable, for example, through the right atrium to the left ventricle (e.g., ventricle-from-atrium, or VfA) or through the coronary sinus. Various non-limiting examples of cardiac therapy include single chamber or multiple chamber pacing (e.g., dual or triple chamber pacing), atrioventricular synchronous pacing, asynchronous pacing, triggered pacing, cardiac resynchronization pacing, or tachycardia-related therapy. In general, a tissue-piercing electrode is implantable in the basal region, septal region, or basal-septal region adjacent to or within the left ventricular myocardium of the patient's heart. The tissue-piercing electrode, or another imageable member, may be at least partially formed of an imageable material.

In one aspect, the present disclosure provides an implantable medical device including one or more electrodes including a tissue-piercing electrode implantable to deliver cardiac therapy to or sense electrical activity of the left ventricle in the basal region, septal region, or basal-septal region of the left ventricular myocardium of a patient's heart. The tissue-piercing electrode is at least partially made of an imageable material. The device also includes a therapy delivery circuit operably coupled to the one or more electrodes to deliver cardiac therapy to the patient's heart; a sensing circuit operably coupled to the one or more electrodes to sense electrical activity of the patient's heart; and a controller including processing circuitry operably coupled to the therapy delivery circuit and the sensing circuit. The controller is configured to: sense electrical activity using one or more electrodes; and deliver cardiac therapy based on the sensed electrical activity.

In another aspect, the present disclosure provides an implantable medical device including one or more electrodes including a tissue-piercing electrode implantable to deliver cardiac therapy to or sense electrical activity of the left ventricle in the basal region, septal region, or basal-septal region of the left ventricular myocardium of a patient's heart. The device also includes an imageable member at least partially made of an imageable material coupled to a housing or a lead configured, when viewed using two-dimensional imaging, to provide orientation information in a third dimension orthogonal to the two dimensions. The device further includes a therapy delivery circuit operably coupled to the one or more electrodes to deliver cardiac therapy to the patient's heart; a sensing circuit operably coupled to the one or more electrodes to sense electrical activity of the patient's heart; and a controller including processing circuitry operably coupled to the therapy delivery circuit and the sensing circuit. The controller is configured to: sense electrical activity using the one or more electrodes; and deliver cardiac therapy based on the sensed electrical activity.

In another non-claimed aspect, the present disclosure provides an implantation method that includes advancing an implantable medical device including a tissue-piercing electrode toward the triangle of Koch region of the right atrium through the right atrial endocardium and central fibrous body or through the coronary sinus to deliver cardiac therapy to or sense electrical activity of the left ventricle in the basal region, septal region, or basal-septal region of the left ventricular myocardium of a patient's heart. The tissue-piercing electrode includes an imageable material or the implantable medical device includes an imageable member at least partially made of an imageable material coupled to a housing or a lead of the implantable medical device. The method also includes acquiring image information representing the patient's heart and the imageable material using two-dimensional imaging; and generating orientation information representing the implantable medical device based on the acquired image information.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

**FIG. 1** is a conceptual diagram of an illustrative cardiac therapy system including an intracardiac medical device implanted in a patient's heart and a separate medical device positioned outside of the patient's heart for use with, e.g., the illustrative methods of **FIGS. 24-25****.**
**FIGS. 2-4** are conceptual diagrams of illustrative cardiac therapy systems including medical devices including leads with electrodes implanted in a patient's heart for use with, e.g., the illustrative methods of **FIGS. 24-25****.**
**FIG. 5** is an enlarged conceptual diagram of the intracardiac medical device of **FIG. 1** and anatomical structures of the patient's heart.
**FIG. 6** is a conceptual diagram of a map of a patient's heart in a standard 17 segment view of the left ventricle showing various electrode implantation locations for use with, e.g., the illustrative systems and devices of **FIGS. 1-4** and **FIG. 16****.**
**FIG. 7** is a perspective view of an intracardiac medical device having a distal fixation and electrode assembly that includes a distal housing-based electrode implemented as a ring electrode for use with, e.g., the illustrative systems and devices of **FIGS. 1-4** and **FIG. 16****.**
**FIG. 8** is a block diagram of illustrative circuitry that may be enclosed within the housing of the medical devices of **FIGS. 1-4** and **FIG. 16**, for example, to provide the functionality and therapy described herein.
**FIG. 9** is a perspective view of another illustrative intracardiac medical device for use with, e.g., the illustrative systems and devices of **FIGS. 1-4** and **FIG. 16****.**
**FIG. 10** is a flowchart of an illustrative method of detecting atrial activity using an atrial motion detector for use with, e.g., the illustrative systems and devices of **FIGS. 1-4** and **FIG. 16****.**
**FIG. 11** is a flowchart of an illustrative method of detecting heart sounds to represent physiological response information for use with, e.g., the illustrative systems and devices of **FIGS. 1-4** and **FIG. 16****.**
**FIG. 12** is a flowchart of an illustrative method of detecting bioimpedance to represent physiological response information for use with, e.g., the illustrative systems and devices of **FIGS. 1-4** and **FIG. 16****.**
**FIG. 13** is a diagram of an illustrative system including electrode apparatus, display apparatus, and computing apparatus for use with, e.g., the illustrative systems and devices of **FIGS. 1-4** and **FIG. 16****.**
**FIGS. 14-15** are diagrams of illustrative external electrode apparatus for measuring torso-surface potentials for use with, e.g., the illustrative systems and devices of **FIGS. 1-4** and **FIG. 16****.**
**FIG. 16** is a diagram showing an overhead view of an environment including a patient and an imaging device for use with, e.g., the illustrative systems and devices of **FIGS. 1-4** and the illustrative methods of **FIGS. 24-25****.**
**FIG. 17** is an illustration showing a two-dimensional image of an imageable material of an implantable medical device at 0 degrees relative to a plane acquired by, e.g., the illustrative imaging device of **FIG. 16****.**
**FIG. 18** is an illustration showing a two-dimensional image of the imageable material of the implantable medical device at +15 degrees relative to a plane acquired by, e.g., the illustrative imaging device of **FIG. 16****.**
**FIG. 19** is an illustration showing a two-dimensional image of the imageable material of the implantable medical device at +30 degrees relative to a plane acquired by, e.g., the illustrative imaging device of **FIG. 16****.**
**FIG. 20** is an illustration showing a two-dimensional image of the imageable material of the implantable medical device at +45 degrees relative to a plane acquired by, e.g., the illustrative imaging device of **FIG. 16****.**
**FIG. 21** is an illustration showing a two-dimensional image of the imageable material of the implantable medical device at -15 degrees relative to a plane acquired by, e.g., the illustrative imaging device of **FIG. 16****.**
**FIG. 22** is an illustration showing a two-dimensional image of the imageable material of the implantable medical device at -30 degrees relative to a plane acquired by, e.g., the illustrative imaging device of **FIG. 16****.**
**FIG. 23** is an illustration showing a two-dimensional image of the imageable material of the implantable medical device at -45 degrees relative to a plane acquired by, e.g., the illustrative imaging device of **FIG. 16****.**
**FIG. 24** is a flow diagram showing one example of a method of delivering an implantable medical device of, e.g., the illustrative systems and devices of **FIGS. 1-4** and **FIG. 16****.**
**FIG. 25** is a flow diagram showing another example of a method of delivering an implantable medical device of, e.g., the illustrative systems and devices of **FIGS. 1-4** and **FIG. 16****.**

### DETAILED DESCRIPTION

The techniques of this disclosure generally relate to delivering implantable medical devices to provide cardiac therapy using the cardiac conduction system or left ventricular myocardium implantable, for example, through the right atrium to the left ventricle (e.g., ventricle-from-atrium, or VfA) or through the coronary sinus. In some embodiments, various techniques described herein may be applied to His bundle or bundle branch pacing applications. Various non-limiting examples of cardiac therapy include single chamber or multiple chamber pacing (e.g., dual or triple chamber pacing), atrioventricular synchronous pacing, asynchronous pacing, triggered pacing, cardiac resynchronization pacing, or tachycardia-related therapy. Although reference is made herein to implantable medical devices, such as a pacemaker or ICD, the methods and processes may be used with any medical devices, systems, or methods related to a patient's heart. Various other applications will become apparent to one of skill in the art having the benefit of the present disclosure.

It may be beneficial to provide an implantable medical device delivery system and technique to locate an electrode for sensing or pacing the left ventricular myocardium or the cardiac conduction system. It may be beneficial to provide an implantable medical device that is free of transvenous leads (e.g., a leadless device). It may also be beneficial to provide an implantable medical device capable of being used for various cardiac therapies, such as single or multiple chamber pacing (e.g., dual- or triple-chamber pacing), atrioventricular synchronous pacing, asynchronous pacing, triggered pacing, cardiac resynchronization pacing, or tachycardia-related therapy. Further, it may be beneficial to provide a technique for delivering a medical device to the triangle of Koch region in the right atrium or to the cardiac conduction system in an accurate and precise manner to facilitate pacing the endocardium and/or the His bundle conduction system.

As used herein, the term "capture" in the context of pacing (e.g., effective capture of the heart from pacing) refers to determining whether a desired response is sensed in response to stimuli, such as sensing desirable electrical activity in response to electrical pulses delivered to a portion of the heart.

As used herein, the term "effective" generally refers to meeting conditions that would be sufficient to a person of ordinary skill in the art for performing a particular function. For example, effective pacing of the left ventricle may result in capture of the left ventricle when electrical or mechanical activity of the left ventricle is sensed and determined to provide cardiac therapy as desired.

The present disclosure provides a technique for implanting a tissue-piercing electrode in the basal region, septal region, or basal-septal region adjacent to or within the left ventricular myocardium of the patient's heart. In particular, the tissue-piercing electrode may be positioned to sense or pace the cardiac conduction system or left ventricular myocardium and be implantable, for example, through the right atrium to the left ventricle or through the coronary sinus. The tissue-piercing electrode, or another imageable member, may be at least partially formed of an imageable material. The technique may include using an imaging device to acquire two-dimensional (2D) imaging data and three-dimensional (3D) information (e.g., orientation information) may be generated from the 2D imaging data that represents the implantable medical device.

In some embodiments, the tissue-piercing electrode may be implanted in the basal region, septal region, or basal-septal region of the left ventricular myocardium of the patient's heart from the triangle of Koch region of the right atrium through the right-atrial endocardium and central fibrous body. In a leadless implantable medical device, the tissue-piercing electrode may leadlessly extend from a distal end region of a housing of the device, and the right-atrial electrode may be leadlessly coupled to the housing (e.g., part of or positioned on the exterior of the housing). The right-atrial motion detector may be within the implantable medical device. In a leaded implantable medical device, one or more of the electrodes may be coupled to the housing using an implantable lead. When the device is implanted, the electrodes may be used to sense electrical activity in one or more atria and/or ventricles of a patient's heart. The motion detector may be used to sense mechanical activity in one or more atria and/or ventricles of the patient's heart. In particular, the activity of the right atrium and the left ventricle may be sensed and, optionally, the activity of the right ventricle may be sensed. In general, electrical or mechanical activity may be sensed, determined, acquired, or monitored using various techniques available to one having ordinary skill in the art who has the benefit of the present disclosure. As used herein, the term "monitoring" generally refers to sensing, acquiring, or receiving data or information that may be used, for example, being processed or stored.

The electrodes may be used to deliver cardiac therapy, such as single-chamber pacing for atrial fibrillation, atrioventricular synchronous pacing for bradycardia, asynchronous pacing, triggered pacing, cardiac resynchronization pacing for ventricular dyssynchrony, anti-tachycardia pacing, or shock therapy. Shock therapy may be initiated by the implantable medical device. A separate medical device, such as an extravascular ICD, which may also be implanted, may be in operative communication with the implantable medical device and may deliver an electrical shock in response to a trigger, such as a signaling pulse (e.g., triggering, signaling, or distinctive electrical pulse) provided by the device.

The present disclosure also provides a non-claimed technique to deliver and implant a medical device, for example, in the triangle of Koch region in the right atrium. Various devices may be used to identify the general location of the triangle of Koch region, which may be described as an implantation site. A flexible lead, or another probe, may be advanced to the potential implantation site and used to identify a precise location for implantation of a medical device, such as an electrode, leadlet, lead, or intracardiac device. In particular, the techniques of the present disclosure may be used to implant a device to provide synchronous pacing to patients with dyssynchrony, as well as provide dual chamber pacing for bradycardia patients with moderate heart failure.

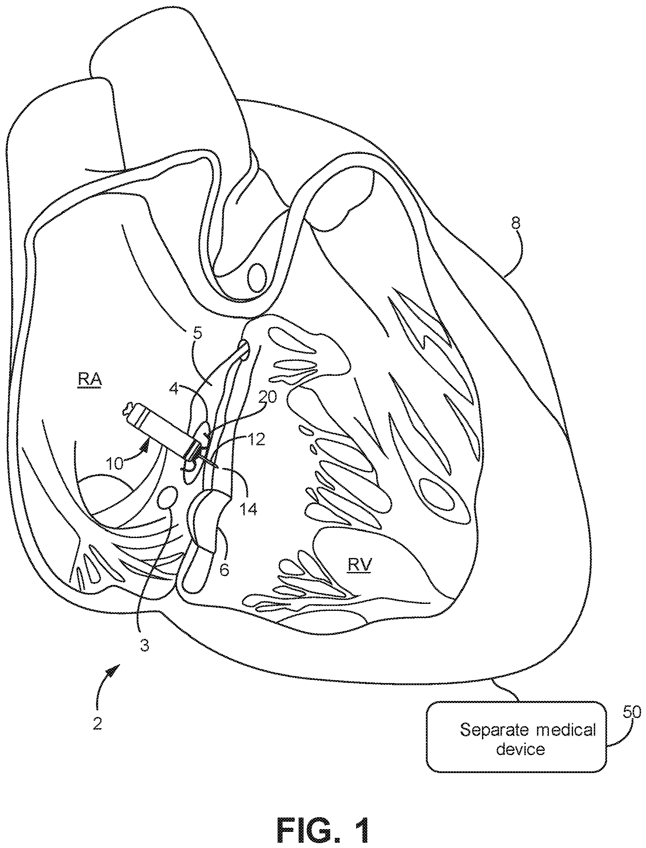

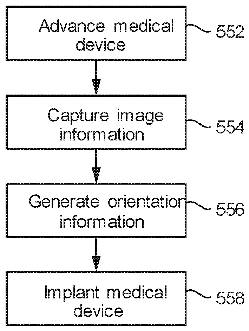

**FIGS. 1-4** show examples of various cardiac therapy systems that may be implanted using, for example, the methods shown in **FIGS. 24-25** to deliver a medical device to an implantation site. In these views, the left ventricle (LV) is positioned generally behind the right ventricle (RV).

Although the present disclosure describes leadless and leaded implantable medical devices, reference is first made to **FIG. 1****,** which shows a conceptual diagram of a cardiac therapy system **2** including an intracardiac medical device **10** that may be configured for single or dual chamber therapy and implanted in a patient's heart **8.** In some embodiments, the device **10** may be configured for single-chamber pacing and may, for example, switch between single-chamber and multiple-chamber pacing (e.g., dual- or triple-chamber pacing). As used herein, "intracardiac" refers to a device configured to be implanted entirely within a patient's heart, for example, to provide cardiac therapy. The device **10** is shown implanted in the right atrium (RA) of the patient's heart **8** in a target implant region **4.** The device **10** may include one or more fixation members **20** that anchor a distal end of the device against the atrial endocardium in a target implant region **4.** The target implant region **4** may lie between the His bundle **5** (or bundle of His) and the coronary sinus **3** and may be adjacent the tricuspid valve **6.** The device **10** may be described as a ventricle-from-atrium (VfA) device, which may sense or provide therapy to one or both ventricles (e.g., right ventricle, left ventricle, or both ventricles, depending on the circumstances) while being generally disposed in the right atrium. In particular, the device **10** may include a tissue-piercing electrode that may be implanted in the basal region, septal region, or basal-septal region of the left ventricular myocardium of the patient's heart from the triangle of Koch region of the right atrium through the right-atrial endocardium and central fibrous body.

The device **10** may be described as a leadless implantable medical device. As used herein, "leadless" refers to a device being free of a lead extending out of the patient's heart **8.** In other words, a leadless device may have a lead that does not extend from outside of the patient's heart to the inside of the patient's heart. Some leadless devices may be introduced through a vein, but once implanted, the device is free of, or may not include, any transvenous lead and may be configured to provide cardiac therapy without using any transvenous lead. Further, a leadless VfA device, in particular, does not use a lead to operably connect to an electrode in the ventricle when a housing of the device is positioned in the atrium. Additionally, a leadless electrode may be coupled to the housing of the medical device without using a lead between the electrode and the housing.

The device **10** may include a dart electrode assembly **12** defining, or having, a straight shaft extending from the distal end region of device **10.** The dart electrode assembly **12** may be placed, or at least configured to be placed, through the atrial myocardium and the central fibrous body and into the ventricular myocardium **14,** or along the ventricular septum, without perforating entirely through the ventricular endocardial or epicardial surfaces. The dart electrode assembly **12** may carry, or include, an electrode at the distal end region of the shaft such that the electrode may be positioned within the ventricular myocardium for sensing ventricular signals and delivering ventricular pulses (e.g., to depolarize the left ventricle to initiate a contraction of the left ventricle). In some examples, the electrode at the distal end region of the shaft is a cathode electrode provided for use in a bipolar electrode pair for pacing and sensing. While the implant region **4** as illustrated may enable one or more electrodes of the dart electrode assembly **12** to be positioned in the ventricular myocardium, it is recognized that a device having the aspects disclosed herein may be implanted at other locations for multiple chamber pacing (e.g., dual- or triple-chamber pacing), single-chamber pacing with multiple chamber sensing, single-chamber pacing and/or sensing, or other clinical therapy and applications as appropriate.

One or more portions, or all, of various components, such as the dart electrode assembly **12** (e.g., tissue-piercing electrode), the one or more fixation members **20,** or any other suitable structure, may be at least partially made, or formed, of an imageable material. Such components may be described as imageable members. One example of an imageable material is a radiopaque material, which may be described as a material that is substantially radiopaque or substantially more radiopaque than other nearby structures during implantation. For example, the imageable material may include radiopaque properties that are visible using x-ray fluoroscopy. Non-limiting examples of imageable material include gold, platinum/iridium, or a polymer loaded with at least 75% tungsten. In some embodiments, the imageable materials include an appropriate radiopaque material such as a metal (e.g., stainless steel, tungsten, gold, platinum iridium, or other appropriate biocompatible materials), radiopaque polymer fibers, radiopaque fill fibers, polymers with radiopaque additives, or the like.

Such imageable materials may assist in resolution, such as a higher contrast, of the image of the imageable member. In general, the imageable member may be high in contrast in an x-ray image compared to other components of the device **10.** The imageable member may be surrounded by materials that are not very radiopaque to facilitate higher contrast for efficient visualization.

Further, the geometry, such as a cross sectional geometry, of the imageable member may assist in resolution of the imageable member. Various cross-sectional geometries include hexagon, round, square, and trapezoid. In some embodiments, the imageable member may include a helical, or coil, shape to facilitate determining the orientation of the device **10.** For example, a thread of a screw or a coiled spring can generally define a helical shape that can define a template for forming the helical shape of the imageable member.

In some embodiments, a tissue-piercing electrode of the dart electrode assembly **12** may be at least partially formed of the imageable material. In some embodiments, the one or more fixation members **20** may be formed of the imageable material. In some embodiments, an imageable member may be disposed on an outer surface of a housing of the device **10** (e.g., using sputtering). In some embodiments, an imageable member may be disposed inside of the housing of the device **10,** for example, on an inner surface of the housing.

It is to be understood that although device **10** is described herein as including a single dart electrode assembly, the device **10** may include more than one dart electrode assembly placed, or configured to be placed, through the atrial myocardium and the central fibrous body, and into the ventricular myocardium **14,** or along the ventricular septum, without perforating entirely through the ventricular endocardial or epicardial surfaces. Additionally, each dart electrode assembly may carry, or include, more than a single electrode at the distal end region, or along other regions (e.g., proximal or central regions), of the shaft.

The cardiac therapy system **2** may also include a separate medical device **50** (depicted diagrammatically in **FIG. 1****),** which may be positioned outside the patient's heart **8** (e.g., subcutaneously) and may be operably coupled to the patient's heart **8** to deliver cardiac therapy thereto. In one example, separate medical device **50** may be an extravascular ICD. In some embodiments, an extravascular ICD may include a defibrillation lead including, or carrying, a defibrillation electrode. A therapy vector may exist between the defibrillation electrode on the defibrillation lead and a housing electrode of the ICD. Further, one or more electrodes of the ICD may also be used for sensing electrical signals related to the patient's heart **8.** The ICD may be configured to deliver shock therapy including one or more defibrillation or cardioversion shocks. For example, if an arrhythmia is sensed, the ICD may send a pulse via the electrical lead wires to shock the heart and restore its normal rhythm. In some examples, the ICD may deliver shock therapy without placing electrical lead wires within the heart or attaching electrical wires directly to the heart (subcutaneous ICDs). Examples of extravascular, subcutaneous ICDs that may be used with the system **2** described herein may be described in U.S. Patent No. 9,278,229 (Reinke et al.).

In the case of shock therapy (e.g., defibrillation shocks provided by the defibrillation electrode of the defibrillation lead), the separate medical device **50** (e.g., extravascular ICD) may include a control circuit that uses a therapy delivery circuit to generate defibrillation shocks having any of a number of waveform properties, including leading-edge voltage, tilt, delivered energy, pulse phases, and the like. The therapy delivery circuit may, for instance, generate monophasic, biphasic, or multiphasic waveforms. Additionally, the therapy delivery circuit may generate defibrillation waveforms having different amounts of energy. For example, the therapy delivery circuit may generate defibrillation waveforms that deliver a total of between approximately 60-80 Joules (J) of energy for subcutaneous defibrillation.

The separate medical device **50** may further include a sensing circuit. The sensing circuit may be configured to obtain electrical signals sensed via one or more combinations of electrodes and to process the obtained signals. The components of the sensing circuit may include analog components, digital components, or a combination thereof. The sensing circuit may, for example, include one or more sense amplifiers, filters, rectifiers, threshold detectors, analog-to-digital converters (ADCs), or the like. The sensing circuit may convert the sensed signals to digital form and provide the digital signals to the control circuit for processing and/or analysis. For example, the sensing circuit may amplify signals from sensing electrodes and may convert the amplified signals to multi-bit digital signals by an ADC, and then provide the digital signals to the control circuit. In one or more embodiments, the sensing circuit may also compare processed signals to a threshold to detect the existence of atrial or ventricular depolarizations (e.g., P- or R-waves) and indicate the existence of the atrial depolarization (e.g., P-waves) or ventricular depolarizations (e.g., R-waves) to the control circuit.

The device **10** and the separate medical device **50** may cooperate to provide cardiac therapy to the patient's heart **8.** For example, the device **10** and the separate medical device **50** may be used to detect tachycardia, monitor tachycardia, and/or provide tachycardia-related therapy. For example, the device **10** may communicate with the separate medical device **50** wirelessly to trigger shock therapy using the separate medical device **50.** As used herein, "wirelessly" refers to an operative coupling or connection without using a metal conductor between the device **10** and the separate medical device **50.** In one example, wireless communication may use a distinctive, signaling, or triggering electrical-pulse provided by the device **10** that conducts through the patient's tissue and is detectable by the separate medical device **50.** In another example, wireless communication may use a communication interface (e.g., an antenna) of the device **10** to provide electromagnetic radiation that propagates through patient's tissue and is detectable, for example, using a communication interface (e.g., an antenna) of the separate medical device **50.**

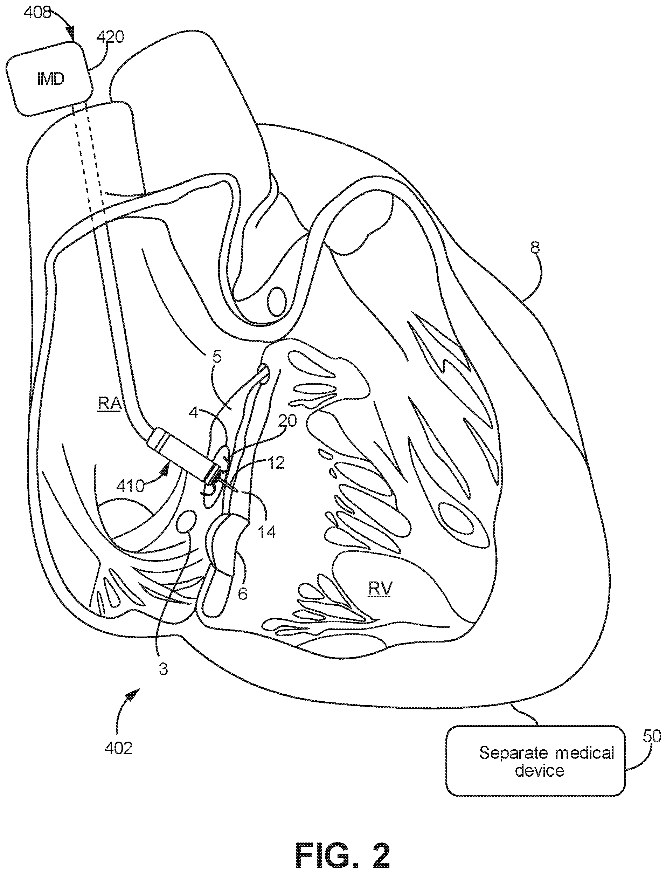

With reference to **FIG. 2****,** a cardiac therapy system **402** may include a leaded medical device **408** including one, or a single, implantable lead **410** having a tissue-piercing electrode assembly **12** coupled to a distal end region of the lead and implanted inside the patient's heart **8.** The housing **420** of the leaded medical device **408** may be implanted and positioned outside of the patient's heart **8** and be configured to calibrate pacing therapy and/or deliver pacing therapy. The lead **410** may include a right-atrial electrode, and the device **408** may operate as a dual-channel capable device (e.g., pacing and/or sensing in both the right atrium and left ventricle). In some embodiments, the lead **410** may not include a right-atrial electrode. In other words, the leaded medical device **408** may be a single channel device, which may be used for asynchronous, triggered, or another type of single-channel pacing. The leaded medical device **408,** using the lead **410,** may sense activity or deliver pacing to the left ventricle (LV) when the tissue-piercing electrode assembly **12** is implanted, for example, in the same or similar manner as described with respect to **FIG. 1****.**

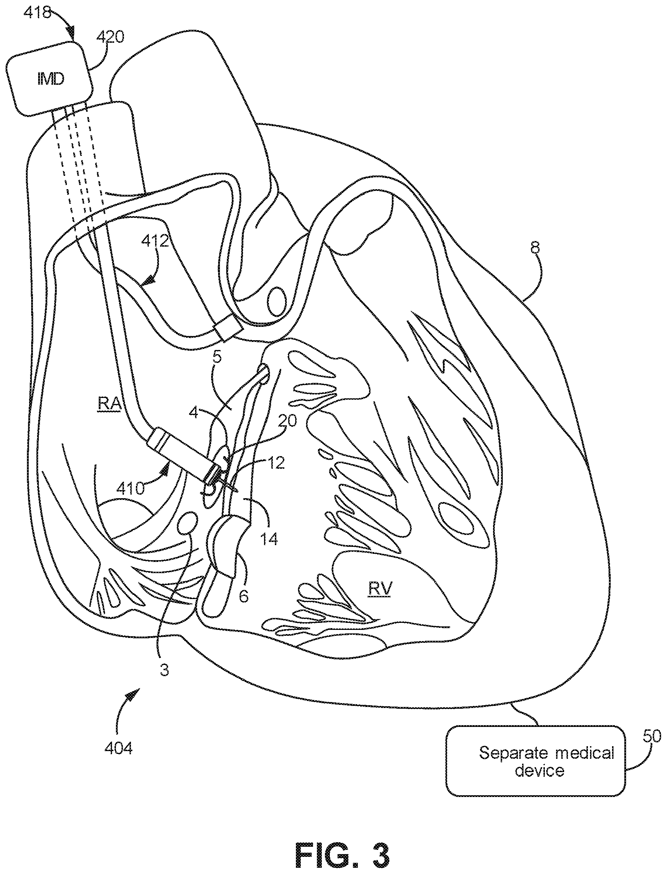

With reference to **FIG. 3****,** a cardiac therapy system **404** may include a leaded medical device **418** similar to the leaded medical device **408** of **FIG. 2****,** except that device **418** includes two implantable leads **410, 412.** In particular, implantable lead **412** may include an electrode (e.g., a right-atrial electrode) coupled to a distal end region of the lead **412** and may be implanted in a different location than lead **410.** In some embodiments, lead **412** is implanted in a different region of the right atrium. In some embodiments, each lead **410, 412** may contribute one channel of a dual-channel device **418.** For example, lead **410** may sense activity or deliver pacing to the left ventricle (LV) when the tissue-piercing electrode of the tissue-piercing electrode assembly **12** is implanted, for example, in the same or similar manner as described with respect to **FIG. 1****,** and lead **412** may sense activity or deliver pacing to the right atrium (RA).

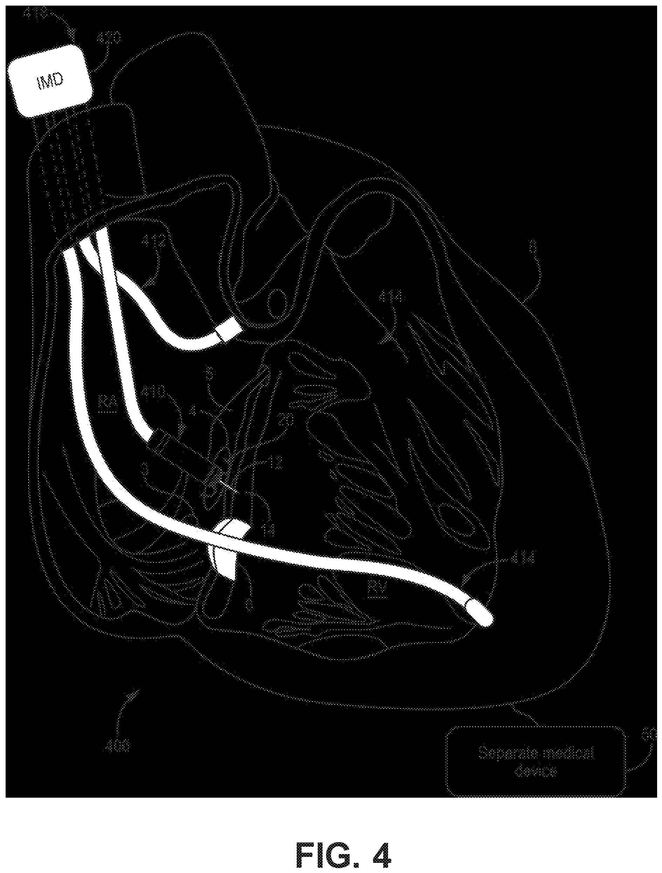

With reference to **FIG. 4****,** a cardiac therapy system **406** may include a leaded medical device **428** similar to the leaded medical device **418** of **FIG. 3** except that device **428** includes three implantable leads **410, 412, 414.** In particular, implantable lead **414** may include an electrode (e.g., a right ventricular electrode) coupled to a distal end region of the lead **414** and may be implanted in a different location than leads **410, 412.** As illustrated, implantable lead **414** extends from the right atrium (RA) to the right ventricle (RV) through tricuspid valve **6.** In some embodiments, lead **414** is implanted in a region of the right ventricle. In some embodiments, each lead **410, 412, 414** may contribute one channel to a multi-channel device **428.** For example, lead **410** may sense activity or deliver pacing to the left ventricle (LV) when the tissue-piercing electrode assembly **12** is implanted, for example, in the same or similar manner as described with respect to **FIG. 1****,** lead **412** may sense activity from the delivery of pacing to the RA, and lead **414** may sense activity or deliver pacing to the RV.

In some embodiments, a pacing delay between the RV electrode on lead **414** to pace the RV and the LV electrode on lead **410** to pace the LV (e.g., RV-LV pacing delay, or more generally, VV pacing delay) may be calibrated or optimized, for example, using a separate medical device, such as an electrode apparatus (e.g., ECG belt). Various methods may be used to calibrate or optimize the VV delay. In some embodiments, the medical device **428** may be used to test pacing at a plurality of different VV delays. For example, the RV may be paced ahead of the LV by about 80, 60, 40, and 20 milliseconds (ms) and the LV may be paced ahead of the RV by about 80, 60, 40, and 20 ms, as well as simultaneous RV-LV pacing (e.g., about 0 ms VV pacing delay). The medical device **428** may then be configured, for example, automatically, to select a VV pacing delay that, when used for pacing, corresponds to a minimal electrical dyssynchrony measured using the electrode apparatus. The test pacing at different VV pacing delays may be performed using a particular AV delay, such as a nominal AV delay set by the medical device **428** or at a predetermined optimal AV delay based on patient characteristics.

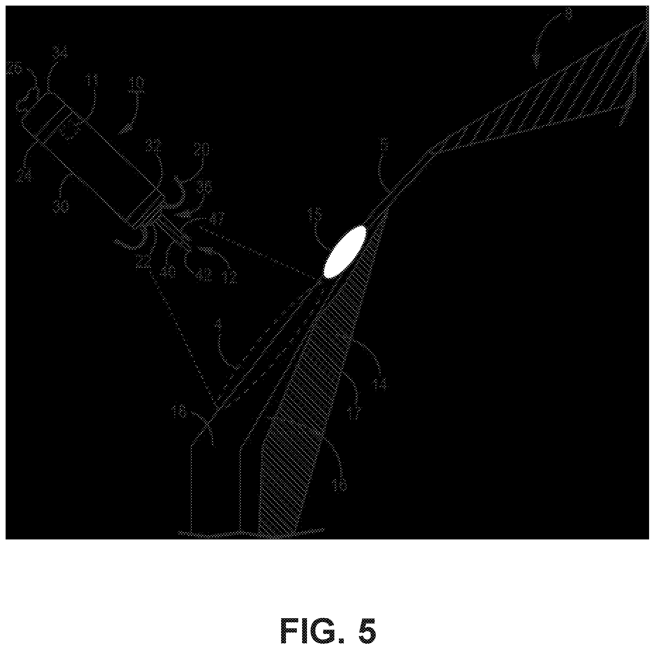

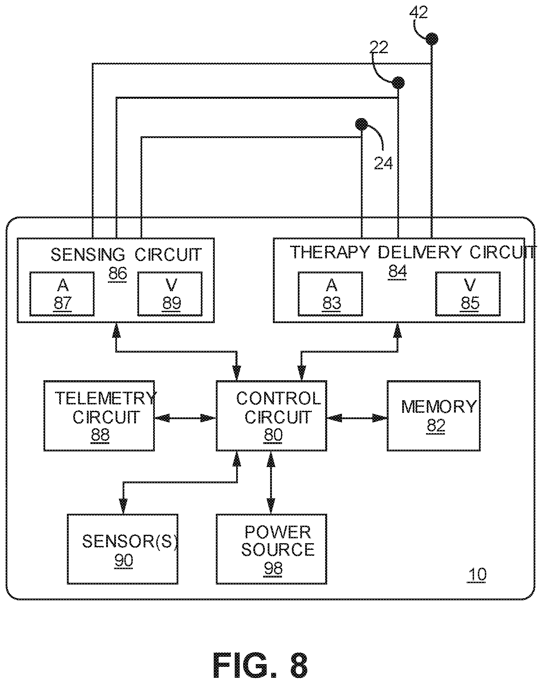

**FIG. 5** is an enlarged conceptual diagram of the intracardiac medical device **10** of **FIG. 1** and anatomical structures of the patient's heart **8.** In particular, the device **10** is configured to sense electrical activity and/or deliver pacing therapy. The intracardiac device **10** may include a housing **30.** The housing **30** may define a hermetically-sealed internal cavity in which internal components of the device **10** reside, such as a sensing circuit, therapy delivery circuit, control circuit, memory, telemetry circuit, other optional sensors, and a power source as generally described in conjunction with **FIG. 8****.** The housing **30** may be formed from an electrically conductive material including titanium or titanium alloy, stainless steel, MP35N (a non-magnetic nickel-cobalt-chromium-molybdenum alloy), platinum alloy, or other bio-compatible metal or metal alloy. In other examples, the housing **30** may be formed from a non-conductive material including ceramic, glass, sapphire, silicone, polyurethane, epoxy, acetyl copolymer plastics, polyether ether ketone (PEEK), a liquid crystal polymer, or other biocompatible polymer.

In at least one embodiment, the housing **30** may be described as extending between a distal end region **32** and a proximal end region **34** in a generally cylindrical shape to facilitate catheter delivery. In other embodiments, the housing **30** may be prismatic or any other shape to perform the functionality and utility described herein. The housing **30** may include a delivery tool interface member **26,** e.g., at the proximal end region **34,** for engaging with a delivery tool during implantation of the device **10.**

All or a portion of the housing **30** may function as an electrode during cardiac therapy, for example, in sensing and/or pacing. In the example shown, the housing-based electrode **24** is shown to circumscribe a proximal portion (e.g., closer to the proximal end region **34** than the distal end region **32)** of the housing **30.** When the housing **30** is formed from an electrically conductive material, such as a titanium alloy or other examples listed above, portions of the housing **30** may be electrically insulated by a non-conductive material, such as a coating of parylene, polyurethane, silicone, epoxy, or other biocompatible polymer, leaving one or more discrete areas of conductive material exposed to define the proximal housing-based electrode **24.** When the housing **30** is formed from a non-conductive material, such as a ceramic, glass or polymer material, an electrically conductive coating or layer, such as a titanium, platinum, stainless steel, or alloys thereof, may be applied to one or more discrete areas of the housing **30** to form the proximal housing-based electrode **24.** In other examples, the proximal housing-based electrode **24** may be a component, such as a ring electrode, that is mounted or assembled onto the housing **30.** The proximal housing-based electrode **24** may be electrically coupled to internal circuitry of the device **10,** e.g., via the electrically-conductive housing **30** or an electrical conductor when the housing **30** is a non-conductive material.

In the example shown, the proximal housing-based electrode **24** is located nearer to the housing proximal end region **34** than the housing distal end region **32** and is therefore referred to as a "proximal housing-based electrode" **24.** In other examples, however, the housing-based electrode **24** may be located at other positions along the housing **30,** e.g., more distal relative to the position shown.

At the distal end region **32,** the device **10** may include a distal fixation and electrode assembly **36,** which may include one or more fixation members **20** and one or more dart electrode assemblies **12** of equal or unequal length. In one example, a single dart electrode assembly **12** includes a shaft **40** extending distally away from the housing distal end region **32** and one or more electrode elements, such as a tip electrode **42** at or near the free, distal end region of the shaft **40.** The tip electrode **42** may have a conical or hemispherical distal tip with a relatively narrow tip-diameter (e.g., less than about 1 millimeter (mm)) for penetrating into and through tissue layers without using a sharpened tip or needle-like tip having sharpened or beveled edges.

The shaft **40** of the dart electrode assembly **12** may be a normally straight member and may be rigid. In other embodiments, the shaft **40** may be described as being relatively stiff but still possessing limited flexibility in lateral directions. Further, the shaft **40** may be non-rigid to allow some lateral flexing with heart motion. However, in a relaxed state, when not subjected to any external forces, the shaft **40** may maintain a straight position as shown to hold the tip electrode **42** spaced apart from the housing distal end region **32** at least by the height **47** of the shaft **40.** In other words, the dart electrode assembly **12** may be described as resilient.

The dart electrode assembly **12** may be configured to pierce through one or more tissue layers to position the tip electrode **42** within a desired tissue layer, e.g., the ventricular myocardium. As such, the height **47,** or length, of the shaft **40** may correspond to the expected pacing site depth, and the shaft **40** may have a relatively high compressive strength along its longitudinal axis to resist bending in a lateral or radial direction when pressed against the implant region **4.** If a second dart electrode assembly **12** is employed, its length may be unequal to the expected pacing site depth and may be configured to act as an indifferent electrode for delivering of pacing energy to the tissue. A longitudinal axial force may be applied against the tip electrode **42,** e.g., by applying a longitudinal pushing force to the proximal end region **34** of the housing **30,** to advance the dart electrode assembly **12** into the tissue within the target implant region. The shaft **40** may be described as longitudinally non-compressive and/or elastically deformable in lateral or radial directions when subjected to lateral or radial forces to allow temporary flexing, e.g., with tissue motion, but may return to its normally straight position when lateral forces diminish. When the shaft **40** is not exposed to any external force, or to only a force along its longitudinal central axis, the shaft **40** may retain a straight, linear position as shown.

The one or more fixation members **20** may be described as one or more "tines" having a normally curved position. The tines may be held in a distally extended position within a delivery tool. The distal tips of tines may penetrate the heart tissue to a limited depth before elastically curving back proximally into the normally curved position (shown) upon release from the delivery tool. Further, the fixation members **20** may include one or more aspects described in, for example, U.S. Patent No. 9,675,579 (Grubac et al.), issued 13 June 2017, and U.S. Patent No. 9,119,959 (Rys et al.), issued 1 September 2015.

In some examples, the distal fixation and electrode assembly **36** includes a distal housing-based electrode **22.** In the case of using the device **10** as a pacemaker for multiple chamber pacing (e.g., dual- or triple-chamber pacing) and sensing, the tip electrode **42** may be used as a cathode electrode paired with the proximal housing-based electrode **24** serving as a return anode electrode. Alternatively, the distal housing-based electrode **22** may serve as a return anode electrode paired with tip electrode **42** for sensing ventricular signals and delivering ventricular pacing pulses. In other examples, the distal housing-based electrode **22** may be a cathode electrode for sensing atrial signals and delivering pacing pulses to the atrial myocardium in the target implant region **4.** When the distal housing-based electrode **22** serves as an atrial cathode electrode, the proximal housing-based electrode **24** may serve as the return anode paired with the tip electrode **42** for ventricular pacing and sensing and as the return anode paired with the distal housing-based electrode **22** for atrial pacing and sensing.

As shown in this illustration, the target implant region **4** in some pacing applications is along the atrial endocardium **18,** generally inferior to the AV node **15** and the His bundle **5.** The dart electrode assembly **12** may at least partially define the height **47,** or length, of the shaft **40** for penetrating through the atrial endocardium **18** in the target implant region **4,** through the central fibrous body **16,** and into the ventricular myocardium **14** without perforating through the ventricular endocardial surface **17.** When the height **47,** or length, of the dart electrode assembly **12** is fully advanced into the target implant region **4,** the tip electrode **42** may rest within the ventricular myocardium **14,** and the distal housing-based electrode **22** may be positioned in intimate contact with or close proximity to the atrial endocardium **18.** The dart electrode assembly **12** may have a total combined height **47,** or length, of the tip electrode **42** and the shaft **40** from about 3 mm to about 8 mm in various examples. The diameter of the shaft **40** may be less than about 2 mm, and may be about 1 mm or less, or even about 0.6 mm or less.

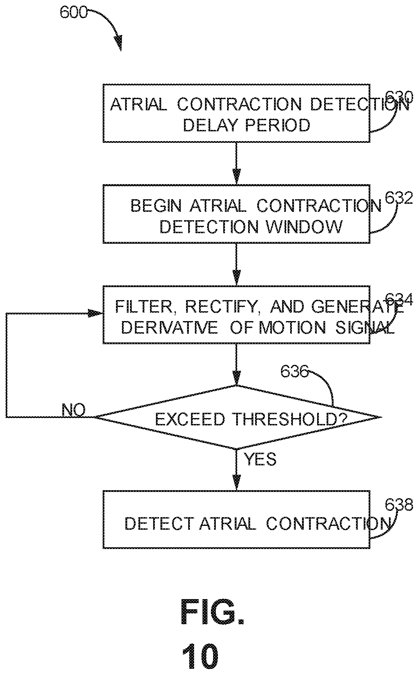

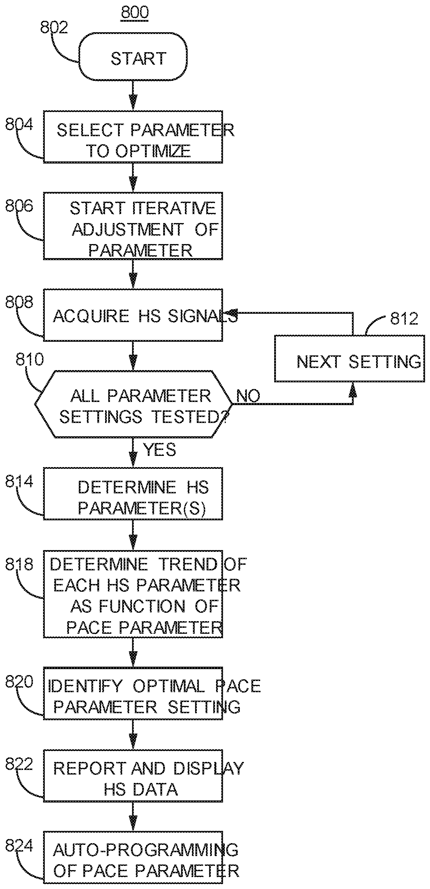

The device **10** may include an acoustic or motion detector **11** within the housing **30.** The acoustic or motion detector **11** may be operably coupled to one or more a control circuit **80 (****FIG. 8****),** a sensing circuit **86 (****FIG. 8****),** or therapy delivery circuit **84 (****FIG. 8****).** In some embodiments, the acoustic or motion detector **11** may be used with methods **600, 650,** or **800** as shown in **FIGS. 10-12****.** The acoustic or motion detector **11** may be used to sense mechanical activity, such as atrial mechanical activity (e.g., an atrial contraction) and/or ventricular mechanical activity (e.g., a ventricular contraction). In some embodiments, the acoustic or motion detector **11** may be used to detect right-atrial mechanical activity. A non-limiting example of an acoustic or motion detector **11** includes an accelerometer or microphone. In some embodiments, the mechanical activity detected by the acoustic or motion detector **11** may be used to supplement or replace electrical activity detected by one or more of the electrodes of the device **10.** For example, the acoustic or motion detector **11** may be used in addition to, or as an alternative to, the proximal housing-based electrode **24.**

The acoustic or motion detector **11** may also be used for rate response detection or to provide a rate-responsive IMD. Various techniques related to rate response may be described in U.S. Patent No. 5,154,170 (Bennett et al.), issued October 13, 1992, entitled "Optimization for rate responsive cardiac pacemaker," and U.S. Patent No. 5,562,711 (Yerich et al.), issued October 8, 1996, entitled "Method and apparatus for rate-responsive cardiac pacing".

In various embodiments, acoustic or motion detector **11** (or motion sensor) may be used as an HS sensor and may be implemented as a microphone or a 1-, 2- or 3-axis accelerometer. In one embodiment, the acoustical sensor is implemented as a piezoelectric crystal mounted within an implantable medical device housing and responsive to the mechanical motion associated with heart sounds. The piezoelectric crystal may be a dedicated HS sensor or may be used for multiple functions. In the illustrative embodiment shown, the acoustical sensor is embodied as a piezoelectric crystal that is also used to generate a patient alert signal in the form of a perceptible vibration of the IMD housing. Upon detecting an alert condition, control circuit **80** may cause patient alert control circuitry to generate an alert signal by activating the piezoelectric crystal.

The control circuit may be used to control whether the piezoelectric crystal is used in a "listening mode" to sense HS signals by HS sensing circuitry or in an "output mode" to generate a patient alert. During patient alert generation, HS sensing circuitry may be temporarily decoupled from the HS sensor by control circuitry.

Examples of other embodiments of acoustical sensors that may be adapted for implementation with the techniques of the present disclosure may be described generally in U.S. Pat. No. 4,546,777 (Groch, et al.), U.S. Pat. No. 6,869,404 (Schulhauser, et al.), U.S. Pat. No. 5,554,177 (Kieval, et al.), and U.S. Pat. No. 7,035,684 (Lee, et al.).

Various types of acoustical sensors may be used. The acoustical sensor may be any implantable or external sensor responsive to one or more of the heart sounds generated as described in the foregoing and thereby produces an analog electrical signal correlated in time and amplitude to the heart sounds. The analog signal may be then be processed, which may include digital conversion, by the HS sensing module to obtain HS parameters, such as amplitudes or relative time intervals, as derived by HS sensing module or control circuit **80.** The acoustical sensor and HS sensing module may be incorporated in an IMD capable of delivering CRT or another cardiac therapy being optimized or may be implemented in a separate device having wired or wireless communication with IMD or an external programmer or computer used during a pace-parameter optimization procedure as described herein.

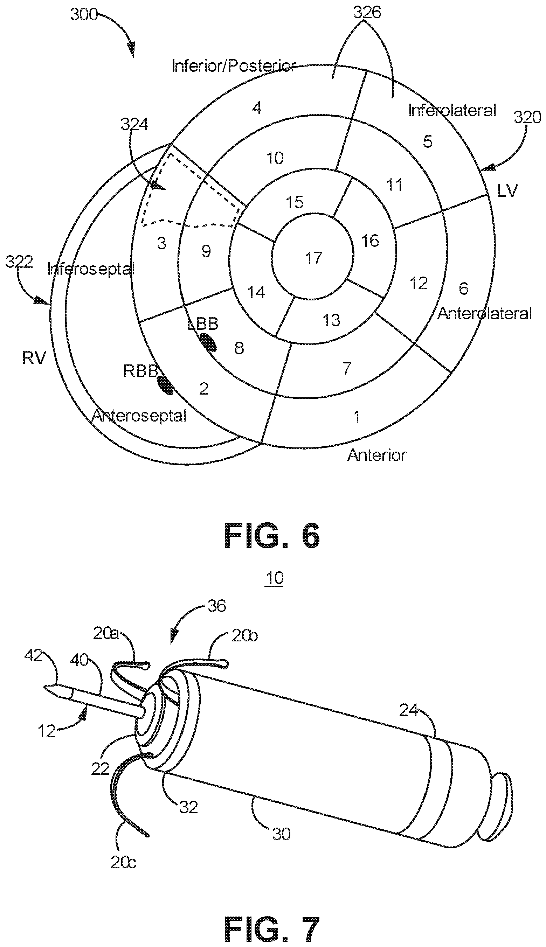

**FIG. 6** is a two-dimensional (2D) ventricular map **300** of a patient's heart (e.g., a top-down view) showing the left ventricle **320** in a standard 17 segment view and the right ventricle **322.** The map **300** includes a plurality of areas **326** corresponding to different regions of a human heart. As illustrated, the areas **326** are numerically labeled 1-17 (e.g., which correspond to 17 segments of the left ventricle of a human heart). Areas **326** of the map **300** may include basal anterior area 1, basal anteroseptal area 2, basal inferoseptal area 3, basal inferior area 4, basal inferolateral area 5, basal anterolateral area 6, mid-anterior area 7, mid-anteroseptal area 8, mid-inferoseptal area 9, mid-inferior area 10, mid-inferolateral area 11, mid-anterolateral area 12, apical anterior area 13, apical septal area 14, apical inferior area 15, apical lateral area 16, and apex area 17. The inferoseptal and anteroseptal areas of the right ventricle **322** are also illustrated, as well as the right bundle branch (RBB) and left bundle branch (LBB).

In some embodiments, any of the tissue-piercing electrodes of the present disclosure may be implanted in the basal region, septal region, or basal-septal region of the left ventricular myocardium of the patient's heart. In particular, the tissue-piercing electrode may be implanted from the triangle of Koch region of the right atrium through the right-atrial endocardium and central fibrous body.

Once implanted, the tissue-piercing electrode may be positioned in the target implant region **4 (****FIGS. 1-5****),** such as the basal region, septal region, or basal-septal region of the left ventricular myocardium. With reference to map **300,** the basal region includes one or more of the basal anterior area 1, basal anteroseptal area 2, basal inferoseptal area 3, basal inferior area 4, mid-anterior area 7, mid-anteroseptal area 8, mid-inferoseptal area 9, and mid-inferior area 10. With reference to map **300,** the septal region includes one or more of the basal anteroseptal area 2, basal anteroseptal area 3, mid-anteroseptal area 8, mid-inferoseptal area 9, and apical septal area 14.

In some embodiments, the tissue-piercing electrode may be positioned in the basal septal region of the left ventricular myocardium when implanted. The basal septal region may include one or more of the basal anteroseptal area 2, basal inferoseptal area 3, mid-anteroseptal area 8, and mid-inferoseptal area 9.

In some embodiments, the tissue-piercing electrode may be positioned in the high inferior/posterior basal septal region of the left ventricular myocardium when implanted. The high inferior/posterior basal septal region of the left ventricular myocardium may include a portion of one or more of the basal inferoseptal area 3 and mid-inferoseptal area 9 (e.g., the basal inferoseptal area only, the mid-inferoseptal area only, or both the basal inferoseptal area and the mid-inferoseptal area). For example, the high inferior/posterior basal septal region may include region **324** illustrated generally as a dashed-line boundary. As shown, the dashed line boundary represents an approximation of where the high inferior/posterior basal septal region is located, which may take a somewhat different shape or size depending on the particular application.

**FIG. 7** is a three-dimensional perspective view of the device **10** capable of calibrating pacing therapy and/or delivering pacing therapy. As shown, the distal fixation and electrode assembly **36** includes the distal housing-based electrode **22** implemented as a ring electrode. The distal housing-based electrode **22** may be positioned in intimate contact with or operative proximity to atrial tissue when fixation member tines **20a**, **20b**, and **20c** of the fixation members **20,** engage with the atrial tissue. The tines **20a**, **20b**, and **20c,** which may be elastically deformable, may be extended distally during delivery of device **10** to the implant site. For example, the tines **20a**, **20b**, and **20c** may pierce the atrial endocardial surface as the device **10** is advanced out of the delivery tool and flex back into their normally curved position (as shown) when no longer constrained within the delivery tool. As the tines **20a**, **20b**, and **20c** curve back into their normal position, the fixation member **20** may pull the distal fixation member and electrode assembly **36** toward the atrial endocardial surface. As the distal fixation member and electrode assembly **36** is pulled toward the atrial endocardium, the tip electrode **42** may be advanced through the atrial myocardium and the central fibrous body and into the ventricular myocardium. The distal housing-based electrode **22** may then be positioned against the atrial endocardial surface.

The distal housing-based electrode **22** may include a ring formed of an electrically conductive material, such as titanium, platinum, iridium, or alloys thereof. The distal housing-based electrode **22** may be a single, continuous ring electrode. In other examples, portions of the ring may be coated with an electrically insulating coating, e.g., parylene, polyurethane, silicone, epoxy, or another insulating coating, to reduce the electrically conductive surface area of the ring electrode. For instance, one or more sectors of the ring may be coated to separate two or more electrically conductive exposed surface areas of the distal housing-based electrode **22.** Reducing the electrically conductive surface area of the distal housing-based electrode **22,** e.g., by covering portions of the electrically conductive ring with an insulating coating, may increase the electrical impedance of the distal housing-based electrode **22,** and thereby, reduce the current delivered during a pacing pulse that captures the myocardium, e.g., the atrial myocardial tissue. A lower current drain may conserve the power source, e.g., one or more rechargeable or non-rechargeable batteries, of the device **10.**

As described above, the distal housing-based electrode **22** may be configured as an atrial cathode electrode for delivering pacing pulses to the atrial tissue at the implant site in combination with the proximal housing-based electrode **24** as the return anode. The electrodes **22** and **24** may be used to sense atrial P-waves for use in controlling atrial pacing pulses (delivered in the absence of a sensed P-wave) and for controlling atrial-synchronized ventricular pacing pulses delivered using the tip electrode **42** as a cathode and the proximal housing-based electrode **24** as the return anode. In other examples, the distal housing-based electrode **22** may be used as a return anode in conjunction with the cathode tip electrode **42** for ventricular pacing and sensing.

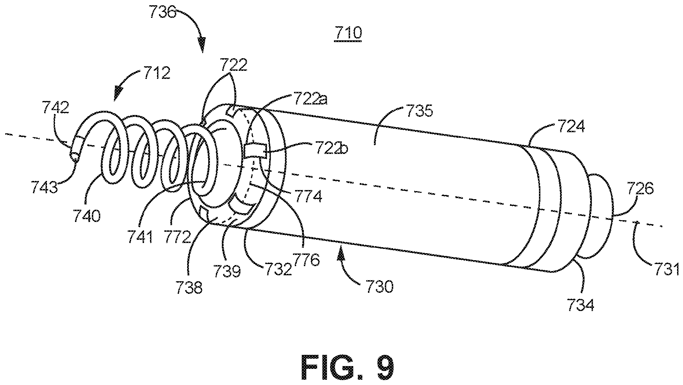

**FIG. 8** is a block diagram of circuitry that may be enclosed within the housing **30 (****FIG. 7****)** to provide the functions of calibrating pacing therapy and/or delivering pacing therapy, using the device **10** according to one example or within the housings of any other medical devices described herein (e.g., device **408** of **FIG. 2****,** device **418** of **FIG. 3****,** device **428** of **FIG. 4****,** or device **710** of **FIG. 9****).** The separate medical device **50 (****FIGS. 1-4****)** may include some or all the same components, which may be configured in a similar manner. The electronic circuitry enclosed within housing **30** may include software, firmware, and hardware that cooperatively acquires atrial and ventricular electrical cardiac signals, determine when a cardiac therapy is necessary, and/or deliver electrical pulses to the patient's heart according to programmed therapy mode and pulse control parameters. The electronic circuitry may include a control circuit **80** (e.g., including processing circuitry), a memory **82,** a therapy delivery circuit **84,** a sensing circuit **86,** and/or a telemetry circuit **88.** In some examples, the device **10** includes one or more sensors **90** for producing a signal that is correlated to a physiological function, state, or condition of the patient, such as a patient activity sensor, for use in determining a need for pacing therapy and/or controlling a pacing rate. For example, one sensor **90** may include an inertial measurement unit (e.g., accelerometer) to measure motion.

The power source **98** may provide power to the circuitry of the device **10** including each of the components **80, 82, 84, 86, 88, 90** as needed. The power source **98** may include one or more energy storage devices, such as one or more rechargeable or non-rechargeable batteries. The connections (not shown) between the power source **98** and each of the components, such as sensors **80, 82, 84, 86, 88, 90,** may be understood from the general block diagram illustrated to one of ordinary skill in the art. For example, the power source **98** may be coupled to one or more charging circuits included in the therapy delivery circuit **84** for providing the power used to charge holding capacitors included in the therapy delivery circuit **84** that are discharged at appropriate times under the control of the control circuit **80** for delivering pacing pulses, e.g., according to a dual chamber pacing mode such as DDI(R). The power source **98** may also be coupled to components of the sensing circuit **86,** such as sense amplifiers, analog-to-digital converters, switching circuitry, etc., sensors **90,** the telemetry circuit **88,** and the memory **82** to provide power to the various circuits.

The functional blocks shown represent functionality included in the device **10** and may include any discrete and/or integrated electronic circuit components that implement analog, and/or digital circuits capable of producing the functions attributed to the medical device **10** herein. The various components may include processing circuitry, such as an application specific integrated circuit (ASIC), an electronic circuit, a processor (shared, dedicated, or group), and memory that execute one or more software or firmware programs, a combinational logic circuit, state machine, or other suitable components or combinations of components that provide the described functionality. The particular form of software, hardware, and/or firmware employed to implement the functionality disclosed herein will be determined primarily by the particular system architecture employed in the medical device and by the particular detection and therapy delivery methodologies employed by the medical device.

The memory **82** may include any volatile, non-volatile, magnetic, or electrical non-transitory computer-readable storage media, such as random-access memory (RAM), read-only memory (ROM), non-volatile RAM (NVRAM), electrically-erasable programmable ROM (EEPROM), flash memory, or any other memory device. Furthermore, the memory **82** may include a non-transitory computer-readable media storing instructions that, when executed by one or more processing circuits, cause the control circuit **80** and/or other processing circuitry to calibrate pacing therapy and/or perform a single, dual, or triple-chamber calibrated pacing therapy (e.g., single or multiple chamber pacing), or other cardiac therapy functions (e.g., sensing or delivering therapy), attributed to the device **10.** The non-transitory computer-readable media storing the instructions may include any of the media listed above.

The control circuit **80** may communicate, e.g., via a data bus, with the therapy delivery circuit **84** and the sensing circuit **86** for sensing cardiac electrical signals and controlling delivery of cardiac electrical stimulation therapies in response to sensed cardiac events, e.g., P-waves and R-waves, or the absence thereof. The tip electrode **42,** the distal housing-based electrode **22,** and the proximal housing-based electrode **24** may be electrically coupled to the therapy delivery circuit **84** for delivering electrical stimulation pulses to the patient's heart and to the sensing circuit **86** and for sensing cardiac electrical signals.

The sensing circuit **86** may include an atrial (A) sensing channel **87** and a ventricular (V) sensing channel **89.** The distal housing-based electrode **22** and the proximal housing-based electrode **24** may be coupled to the atrial sensing channel **87** for sensing atrial signals, e.g., P-waves attendant to the depolarization of the atrial myocardium. In examples that include two or more selectable distal housing-based electrodes, the sensing circuit **86** may include switching circuitry for selectively coupling one or more of the available distal housing-based electrodes to cardiac event detection circuitry included in the atrial sensing channel **87.** Switching circuitry may include a switch array, switch matrix, multiplexer, or any other type of switching device suitable to selectively couple components of the sensing circuit **86** to selected electrodes. The tip electrode **42** and the proximal housing-based electrode **24** may be coupled to the ventricular sensing channel **89** for sensing ventricular signals, e.g., R-waves attendant to the depolarization of the ventricular myocardium.

Each of the atrial sensing channel **87** and the ventricular sensing channel **89** may include cardiac event detection circuitry for detecting P-waves and R-waves, respectively, from the cardiac electrical signals received by the respective sensing channels. The cardiac event detection circuitry included in each of the channels **87** and **89** may be configured to amplify, filter, digitize, and rectify the cardiac electrical signal received from the selected electrodes to improve the signal quality for detecting cardiac electrical events. The cardiac event detection circuitry within each channel **87** and **89** may include one or more sense amplifiers, filters, rectifiers, threshold detectors, comparators, analog-to-digital converters (ADCs), timers, or other analog or digital components. A cardiac event sensing threshold, e.g., a P-wave sensing threshold and an R-wave sensing threshold, may be automatically adjusted by each respective sensing channel **87** and **89** under the control of the control circuit **80,** e.g., based on timing intervals and sensing threshold values determined by the control circuit **80,** stored in the memory **82,** and/or controlled by hardware, firmware, and/or software of the control circuit **80** and/or the sensing circuit **86.**

Upon detecting a cardiac electrical event based on a sensing threshold crossing, the sensing circuit **86** may produce a sensed event signal that is passed to the control circuit **80.** For example, the atrial sensing channel **87** may produce a P-wave sensed event signal in response to a P-wave sensing threshold crossing. The ventricular sensing channel **89** may produce an R-wave sensed event signal in response to an R-wave sensing threshold crossing. The sensed event signals may be used by the control circuit **80** for setting pacing escape interval timers that control the basic time intervals used for scheduling cardiac pacing pulses. A sensed event signal may trigger or inhibit a pacing pulse depending on the particular programmed pacing mode. For example, a P-wave sensed event signal received from the atrial sensing channel **87** may cause the control circuit **80** to inhibit a scheduled atrial pacing pulse and schedule a ventricular pacing pulse at a programmed atrioventricular (AV) pacing interval. If an R-wave is sensed before the AV pacing interval expires, the ventricular pacing pulse may be inhibited. If the AV pacing interval expires before the control circuit **80** receives an R-wave sensed event signal from the ventricular sensing channel **89,** the control circuit **80** may use the therapy delivery circuit **84** to deliver the scheduled ventricular pacing pulse synchronized to the sensed P-wave.

In some examples, the device **10** may be configured to deliver a variety of pacing therapies including bradycardia pacing, cardiac resynchronization therapy, post-shock pacing, and/or tachycardia-related therapy, such as ATP, among others. For example, the device **10** may be configured to detect non-sinus tachycardia and deliver ATP. The control circuit **80** may determine cardiac event time intervals, e.g., P-P intervals between consecutive P-wave sensed event signals received from the atrial sensing channel **87,** R-R intervals between consecutive R-wave sensed event signals received from the ventricular sensing channel **89,** and P-R and/or R-P intervals received between P-wave sensed event signals and R-wave sensed event signals. These intervals may be compared to tachycardia detection intervals for detecting non-sinus tachycardia. Tachycardia may be detected in a given heart chamber based on a threshold number of tachycardia detection intervals being detected.

The therapy delivery circuit **84** may include atrial pacing circuit **83** and ventricular pacing circuit **85.** Each pacing circuit **83, 85** may include charging circuitry, one or more charge storage devices such as one or more low voltage holding capacitors, an output capacitor, and/or switching circuitry that controls when the holding capacitor(s) are charged and discharged across the output capacitor to deliver a pacing pulse to the pacing electrode vector coupled to respective pacing circuits **83, 85.** The tip electrode **42** and the proximal housing-based electrode **24** may be coupled to the ventricular pacing circuit **85** as a bipolar cathode and anode pair for delivering ventricular pacing pulses, e.g., upon expiration of an AV or VV pacing interval set by the control circuit **80** for providing atrial-synchronized ventricular pacing and a basic lower ventricular pacing rate.

The atrial pacing circuit **83** may be coupled to the distal housing-based electrode **22** and the proximal housing-based electrode **24** to deliver atrial pacing pulses. The control circuit **80** may set one or more atrial pacing intervals according to a programmed lower pacing rate or a temporary lower rate set according to a rate-responsive sensor-indicated pacing rate. Atrial pacing circuit may be controlled to deliver an atrial pacing pulse if the atrial pacing interval expires before a P-wave sensed event signal is received from the atrial sensing channel **87.** The control circuit **80** starts an AV pacing interval in response to a delivered atrial pacing pulse to provide synchronized multiple chamber pacing (e.g., dual- or triple-chamber pacing).

Charging of a holding capacitor of the atrial or ventricular pacing circuit **83, 85** to a programmed pacing voltage amplitude and discharging of the capacitor for a programmed pacing pulse width may be performed by the therapy delivery circuit **84** according to control signals received from the control circuit **80.** For example, a pace timing circuit included in the control circuit **80** may include programmable digital counters set by a microprocessor of the control circuit **80** for controlling the basic pacing time intervals associated with various single-chamber or multiple-chamber pacing (e.g., dual- or triple- chamber pacing) modes or anti-tachycardia pacing sequences. The microprocessor of the control circuit **80** may also set the amplitude, pulse width, polarity, or other characteristics of the cardiac pacing pulses, which may be based on programmed values stored in the memory **82.**

The device **10** may include other sensors **90** for sensing signals from the patient for use in determining a need for and/or controlling electrical stimulation therapies delivered by the therapy delivery circuit **84.** In some examples, a sensor indicative of a need for increased cardiac output may include a patient activity sensor, such as an accelerometer. An increase in the metabolic demand of the patient due to increased activity as indicated by the patient activity sensor may be determined by the control circuit **80** for use in determining a sensor-indicated pacing rate.

Control parameters utilized by the control circuit **80** for sensing cardiac events and controlling pacing therapy delivery may be programmed into the memory **82** via the telemetry circuit **88,** which may also be described as a communication interface. The telemetry circuit **88** includes a transceiver and antenna for communicating with an external device, such as a programmer or home monitor, using radio frequency communication or other communication protocols. The control circuit **80** may use the telemetry circuit **88** to receive downlink telemetry from and send uplink telemetry to the external device. In some cases, the telemetry circuit **88** may be used to transmit and receive communication signals to/from another medical device implanted in the patient.

**FIG. 9** is a three-dimensional perspective view of another leadless intracardiac medical device **710** that may be configured for calibrating pacing therapy and/or delivering pacing therapy for single or multiple chamber cardiac therapy (e.g., dual- or triple-chamber cardiac therapy) according to another example. The device **710** may include a housing **730** having an outer sidewall **735,** shown as a cylindrical outer sidewall, extending from a housing distal end region **732** to a housing proximal end region **734.** The housing **730** may enclose electronic circuitry configured to perform single- or multiple-chamber cardiac therapy, including atrial and ventricular cardiac electrical signal sensing and pacing the atrial and ventricular chambers. The delivery tool interface member **726** is shown on the housing proximal end region **734.**

A distal fixation and electrode assembly **736** may be coupled to the housing distal end region **732.** The distal fixation and electrode assembly **736** may include an electrically insulative distal member **772** coupled to the housing distal end region **732.** The tissue-piercing electrode assembly **712** extends away from the housing distal end region **732,** and multiple non-tissue piercing electrodes **722** may be coupled directly to the insulative distal member **772.** The tissue-piercing electrode assembly **712** extends in a longitudinal direction away from the housing distal end region **732** and may be coaxial with the longitudinal center axis **731** of the housing **730.**

The distal tissue-piercing electrode assembly **712** may include an electrically insulated shaft **740** and a tip electrode **742** (e.g., tissue-piercing electrode). In some examples, the tissue-piercing electrode assembly **712** is an active fixation member including a helical shaft **740** and a distal cathode tip electrode **742.** The helical shaft **740** may extend from a shaft distal end region **743** to a shaft proximal end region **741,** which may be directly coupled to the insulative distal member **772.** The helical shaft **740** may be coated with an electrically insulating material, e.g., parylene or other examples listed herein, to avoid sensing or stimulation of cardiac tissue along the shaft length. The tip electrode **742** is at the shaft distal end region **743** and may serve as a cathode electrode for delivering ventricular pacing pulses and sensing ventricular electrical signals using the proximal housing-based electrode **724** as a return anode when the tip electrode **742** is advanced into ventricular tissue. The proximal housing-based electrode **724** may be a ring electrode circumscribing the housing **730** and may be defined by an uninsulated portion of the longitudinal sidewall **735.** Other portions of the housing **730** not serving as an electrode may be coated with an electrically insulating material as described above in conjunction with **FIG. 7****.**

Using two or more tissue-piercing electrodes (e.g., of any type) penetrating into the LV myocardium may be used for more localized pacing capture and may mitigate ventricular pacing spikes affecting capturing atrial tissue. In some embodiments, multiple tissue-piercing electrodes may include two or more dart-type electrode assemblies (e.g., electrode assembly **12** of **FIG. 7****),** a helical-type electrode (e.g., electrode assembly **712)** Non-limiting examples of multiple tissue-piercing electrodes include two dart electrode assemblies, a helix electrode with a dart electrode assembly extending therethrough (e.g., through the center), or dual intertwined helixes. Multiple tissue-piercing electrodes may also be used for bipolar or multi-polar pacing.

In some embodiments, one or more tissue-piercing electrodes (e.g., of any type) that penetrate into the LV myocardium may be a multi-polar tissue-piercing electrode. A multi-polar tissue-piercing electrode may include one or more electrically active and electrically separate elements, which may enable bipolar or multi-polar pacing from one or more tissue-piercing electrodes.

Multiple non-tissue piercing electrodes **722** may be provided along a periphery of the insulative distal member **772,** peripheral to the tissue-piercing electrode assembly **712.** The insulative distal member **772** may define a distal-facing surface **738** of the device **710** and a circumferential surface **739** that circumscribes the device **710** adj acent to the housing longitudinal sidewall **735.** Non-tissue piercing electrodes **722** may be formed of an electrically conductive material, such as titanium, platinum, iridium, or alloys thereof. In the illustrated embodiment, six non-tissue piercing electrodes **722** are spaced apart radially at equal distances along the outer periphery of insulative distal member **772.** However, two or more non-tissue piercing electrodes **722** may be provided.

Non-tissue piercing electrodes **722** may be discrete components each retained within a respective recess **774** in the insulative member **772** sized and shaped to mate with the non-tissue piercing electrode **722.** In other examples, the non-tissue piercing electrodes **722** may each be an uninsulated, exposed portion of a unitary member mounted within or on the insulative distal member **772.** Intervening portions of the unitary member not functioning as an electrode may be insulated by the insulative distal member **772** or, if exposed to the surrounding environment, may be coated with an electrically insulating coating, e.g., parylene, polyurethane, silicone, epoxy, or another insulating coating.

When the tissue-piercing electrode assembly **712** is advanced into cardiac tissue, at least one non-tissue piercing electrode **722** may be positioned against, in intimate contact with, or in operative proximity to, a cardiac tissue surface for delivering pulses and/or sensing cardiac electrical signals produced by the patient's heart. For example, non-tissue piercing electrodes **722** may be positioned in contact with right-atrial endocardial tissue for pacing and sensing in the atrium when the tissue-piercing electrode assembly **712** is advanced into the atrial tissue and through the central fibrous body until the distal tip electrode **742** is positioned in direct contact with ventricular tissue, e.g., ventricular myocardium and/or a portion of the ventricular conduction system.

Non-tissue piercing electrodes **722** may be coupled to the therapy delivery circuit **84** and the sensing circuit **86** (see **FIG. 8****)** enclosed by the housing **730** to function collectively as a cathode electrode for delivering atrial pacing pulses and for sensing atrial electrical signals, e.g., P-waves, in combination with the proximal housing-based electrode **724** as a return anode. Switching circuitry included in the sensing circuit **86** may be activated under the control of the control circuit **80** to couple one or more of the non-tissue piercing electrodes to the atrial sensing channel **87.** Distal, non-tissue piercing electrodes **722** may be electrically isolated from each other so that each individual one of the electrodes **722** may be individually selected by switching circuitry included in the therapy delivery circuit **84** to serve alone or in a combination of two or more of the electrodes **722** as an atrial cathode electrode. Switching circuitry included in the therapy delivery circuit **84** may be activated under the control of the control circuit **80** to couple one or more of the non-tissue piercing electrodes **722** to the atrial pacing circuit **83.** Two or more of the non-tissue piercing electrodes **722** may be selected at a time to operate as a multi-point atrial cathode electrode.

Certain non-tissue piercing electrodes **722** selected for atrial pacing and/or atrial sensing may be selected based on atrial capture threshold tests, electrode impedance, P-wave signal strength in the cardiac electrical signal, or other factors. For example, a single one or any combination of two or more individual non-tissue piercing electrodes **722** functioning as a cathode electrode that provides an optimal combination of a low pacing capture threshold amplitude and relatively high electrode impedance may be selected to achieve reliable atrial pacing using minimal current drain from the power source **98.**

In some instances, the distal-facing surface **738** may uniformly contact the atrial endocardial surface when the tissue-piercing electrode assembly **712** anchors the housing **730** at the implant site. In that case, all the electrodes **722** may be selected together to form the atrial cathode. Alternatively, every other one of the electrodes **722** may be selected together to form a multi-point atrial cathode having a higher electrical impedance that is still uniformly distributed along the distal-facing surface **738.** Alternatively, a subset of one or more electrodes **722** along one side of the insulative distal member **772** may be selected to provide pacing at a desired site that achieves the lowest pacing capture threshold due to the relative location of the electrodes **722** to the atrial tissue being paced.

In other instances, the distal-facing surface **738** may be oriented at an angle relative to the adjacent endocardial surface depending on the positioning and orientation at which the tissue-piercing electrode assembly **712** enters the cardiac tissue. In this situation, one or more of the non-tissue piercing electrodes **722** may be positioned in contact with the adjacent endocardial tissue closer than other non-tissue piercing electrodes **722,** which may be angled away from the endocardial surface. By providing multiple non-tissue piercing electrodes along the periphery of the insulative distal member **772,** the angle of the tissue-piercing electrode assembly **712** and the housing distal end region **732** relative to the cardiac surface, e.g., the right-atrial endocardial surface, may not be required to be substantially parallel. Anatomical and positional differences may cause the distal-facing surface **738** to be angled or oblique to the endocardial surface, however, multiple non-tissue piercing electrodes **722** distributed along the periphery of the insulative distal member **772** increase the likelihood of good contact between one or more electrodes **722** and the adjacent cardiac tissue to promote acceptable pacing thresholds and reliable cardiac event sensing using at least a subset of multiple electrodes **722.** Contact or fixation circumferentially along the entire periphery of the insulative distal member **772** may not be required.

The non-tissue piercing electrodes **722** are shown to each include a first portion **722a** extending along the distal-facing surface **738** and a second portion **722b** extending along the circumferential surface **739.** The first portion **722a** and the second portion **722b** may be continuous, exposed surfaces such that the active electrode surface wraps around a peripheral edge **776** of the insulative distal member **772** that joins the distal facing surface **738** and the circumferential surface **739.** The non-tissue piercing electrodes **722** may include one or more of the electrodes **722** along the distal-facing surface **738,** one or more electrodes along the circumferential surface **739,** one or more electrodes each extending along both of the distal-facing surface **738** and the circumferential surface **739,** or any combination thereof. The exposed surface of each of the non-tissue piercing electrodes **722** may be flush with respective distal-facing surfaces **738** and/or circumferential surfaces. In other examples, each of the non-tissue piercing electrodes **722** may have a raised surface that protrudes from the insulative distal member **772.** Any raised surface of the electrodes **722,** however, may define a smooth or rounded, non-tissue piercing surface.

The distal fixation and electrode assembly **736** may seal the distal end region of the housing **730** and may provide a foundation on which the electrodes **722** are mounted. The electrodes **722** may be referred to as housing-based electrodes. The electrodes **722** may not be carried by a shaft or other extension that extends the active electrode portion away from the housing **730,** like the distal tip electrode **742** residing at the distal tip of the helical shaft **740** extending away from the housing **730.** Other examples of non-tissue piercing electrodes presented herein that are coupled to a distal-facing surface and/or a circumferential surface of an insulative distal member include the distal housing-based ring electrode **22 (****FIG. 7****),** the distal housing-based ring electrode extending circumferentially around the assembly **36 (****FIG. 7****),** button electrodes, other housing-based electrodes, and other circumferential ring electrodes. Any non-tissue piercing electrodes directly coupled to a distal insulative member, peripherally to a central tissue-piercing electrode, may be provided to function individually, collectively, or in any combination as a cathode electrode for delivering pacing pulses to adjacent cardiac tissue. When a ring electrode, such as the distal ring electrode **22** and/or a circumferential ring electrode, is provided, portions of the ring electrode may be electrically insulated by a coating to provide multiple distributed non-tissue piercing electrodes along the distal-facing surface and/or the circumferential surface of the insulative distal member.

The non-tissue piercing electrodes **722** and other examples listed above are expected to provide more reliable and effective atrial pacing and sensing than a tissue-piercing electrode provided along the distal fixation and electrode assembly **736.** The atrial chamber walls are relatively thin compared to ventricular chamber walls. A tissue-piercing atrial cathode electrode may extend too deep within the atrial tissue leading to inadvertent sustained or intermittent capture of ventricular tissue. A tissue-piercing atrial cathode electrode may lead to interference with sensing atrial signals due to ventricular signals having a larger signal strength in the cardiac electrical signal received via tissue-piercing atrial cathode electrodes that are closer in physical proximity to the ventricular tissue. The tissue-piercing electrode assembly **712** may be securely anchored into ventricular tissue for stabilizing the implant position of the device **710** and providing reasonable certainty that the tip electrode **742** is sensing and pacing in ventricular tissue while the non-tissue piercing electrodes **722** are reliably pacing and sensing in the atrium. When the device **710** is implanted in the target implant region **4,** e.g., as shown in **FIG. 1** the ventricular septum, the tip electrode **742** may reach left ventricular tissue for pacing of the left ventricle while the non-tissue piercing electrodes **722** provide pacing and sensing in the right atrium. The tissue-piercing electrode assembly **712** may be in the range of about 4 to about 8 mm in length from the distal-facing surface **738** to reach left ventricular tissue. In some instances, the device **710** may achieve four-chamber pacing by delivering atrial pacing pulses from the atrial pacing circuit **83** via the non-tissue piercing electrodes **722** in the target implant region **4** to achieve bi-atrial (right and left atrial) capture and by delivering ventricular pacing pulses from the ventricular pacing circuit **85** via the tip electrode **742** advanced into ventricular tissue from the target implant region **4** to achieve biventricular (right and left ventricular) capture.

**FIG. 10** shows an illustrative method **600** of detecting atrial activity, for example, using the acoustic or motion detector **11** of **FIG. 5****,** which may be used to represent physiological response information. In particular, method **600** may include detecting an atrial contraction based on analysis of a motion signal (e.g., provided by the motion detector **11)** that may be performed by an IMD implanted in the patient's heart. In some embodiments, the motion signal may be provided by an IMD implanted within a ventricle, such as the right ventricle, of the patient's heart. The method **600** may include beginning an atrial contraction detection delay period upon identification of a ventricular activation event **630.** The method **600** may include beginning an atrial contraction detection window upon expiration of the atrial contraction delay period **632.** The method **600** may include analyzing the motion signal within the atrial contraction detection window.

The method **600** may include filtering the motion signal within the atrial contraction detection window, rectifying the filtered signal, and generating a derivative signal of the filtered and rectified motion signal **634** within the atrial contraction detection window. The method **600** may include determining whether an amplitude of the derivative signal within the atrial contraction detection window exceeds a threshold **636.** In response to determining that the amplitude of the derivative signal within the atrial contraction detection window exceeds the threshold (YES of **636),** the method **600** may proceed to detecting atrial contraction **638.** Otherwise (NO of **636),** the method **600** may return to filtering, rectifying, and generating a derivative signal **634.** Various techniques for using a motion detector that provides a motion signal may be described in U.S. Patent No. 9,399,140 (Cho et al.), issued July 26, 2016, entitled "Atrial contraction detection by a ventricular leadless pacing device for atrio-synchronous ventricular pacing."

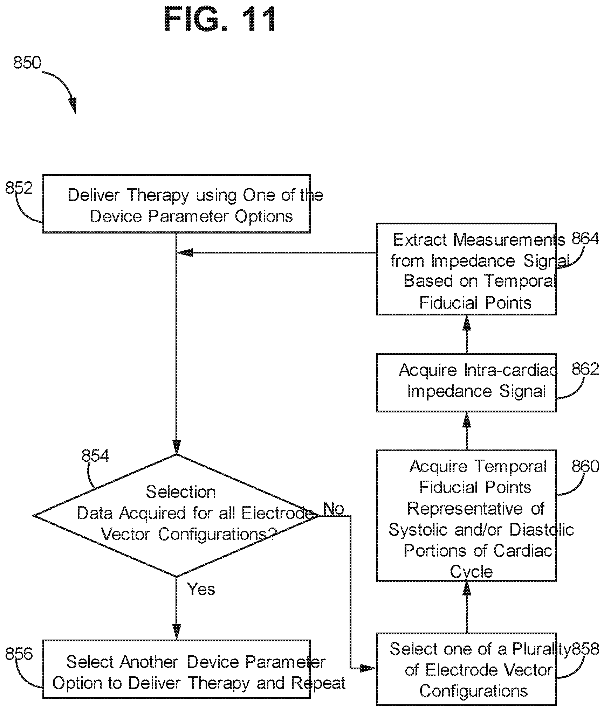

As will be described with respect to **FIG. 11****,** heart sounds (HS) may be detected and used to represent physiological response information, described herein, the amplitudes and/or relative time intervals of one or more of the S1 through S4 heart sounds can be useful in optimizing a patient's hemodynamic response to CRT or other cardiac therapies that include cardiac pacing and/or neural stimulation for achieving hemodynamic benefit. The first heart sound, S1, corresponds to the start of ventricular systole. Ventricular systole begins when an action potential conducts through the atrioventricular node (AV node) and quickly depolarizes the ventricular myocardium. This event is distinguished by the QRS complex on the ECG. As the ventricles contract, the pressure in the ventricles begins to rise, causing abrupt closure of the mitral and tricuspid valves between the ventricles and atria as ventricular pressure exceeds atrial pressure. This valve closure may generate S1. S1 generally has a duration of about 150 ms and a frequency on the order of about 20 to 250 Hz. The amplitude of S1 may provide a surrogate measurement of LV contractility. Thus, an increase in S 1 amplitude positively may correlate with an improvement in LV contractility. Other measures, like the pre-ejection period measured from the onset of QRS to S 1, may also be used as a surrogate of myocardial contractility index.

Separation of the closure of the mitral and tricuspid valves due to ventricular dyssynchrony can be observed as separate M1 and T1 peaks in the S1 signal. Merging of the M1 (mitral valve closure sound) and the T1 (tricuspid valve closure sound) can be used as an indication of improved ventricular synchrony.

Generally, left ventricular pressure (LVP) rises dramatically following the QRS complex of the ECG and closure of the mitral valve and continues to build during ventricular systole until the aortic and pulmonary valves open, ejecting blood into the aorta and pulmonary artery. Ventricular contraction typically continues to cause blood pressure to rise in the ventricles and the aorta and pulmonary artery during the ejection phase. As the contraction diminishes, blood pressure decreases until the aortic and pulmonary valves close.

The second heart sound, S2, may be generated by the closure of the aortic and pulmonary valves, near the end of ventricular systole and start of ventricular diastole. S2 may, therefore, be correlated to diastolic pressure in the aorta and the pulmonary artery. S2 generally has a duration of about 120 ms and a frequency on the order of 25 to 350 Hz. The time interval between S 1 and S2, i.e., S 1-S2 time interval may represent the systolic time interval (STI) corresponding to the ventricular isovolumic contraction (pre-ejection) and ejection phase of the cardiac cycle. This S1-S2 time interval may provide a surrogate measurement for stroke volume. Furthermore, the ratio of the pre-ejection period (Q-S1) to S 1-S2 time may be used as an index of myocardial contractility.

The third heart sound, S3, is associated with early, passive diastolic filling of the ventricles, and the fourth heart sound, S4, may be associated with late, active filling of the ventricles due to atrial contraction. The third sound is generally difficult to hear in a normal patient using a stethoscope, and the fourth sound is generally not heard in a normal patient. Presence of the third and fourth heart sounds during an examination using a stethoscope may indicate a pathological condition. The S3 and S4 heart sounds may be used in optimizing pace parameters as they relate to the diastolic function of the heart. Generally, these sounds would be minimized or disappear when an optimal pace parameter is identified. Other aspects of the S1 through S4 heart sounds and timing thereof that may be useful in cardiac pace-parameter optimization as known to one having ordinary skill in the art.

**FIG. 11** is a flowchart **800** of a non-claimed method for using heart sounds to optimize pace control parameters according to one embodiment. Methods of the present disclosure may include one or more blocks shown in flowchart **800.** Other examples of using heart sounds to optimize cardiac therapy are described generally in U.S. Patent No. 9,643,0134, granted May 9, 2017, entitled "System and method for pacing parameter optimization using heart sounds."

A pace-parameter optimization method may be initiated at block **802.** The optimization process may be initiated in response to a user command received via an external programmer. At a time of initial IMD implantation or during office follow-up visits, or during a remote patient monitoring session, a user may initiate an HS-base optimization procedure using an external programmer or networked computer. Additionally, or alternatively, the process shown by flowchart **800** may be an automated process started periodically or in response to sensing a need for therapy delivery or therapy adjustment based on a sensed physiological signal, which may include sensed HS signals.

At block **804** a pace control parameter to be optimized is selected. A control parameter may be a timing-related parameter, such as an AV interval or VV interval. Pacing vector is another control parameter that may be selected at block **804** for optimization. For example, when a multi-polar lead is used, such as a coronary sinus lead, multiple bipolar or unipolar pacing vectors may be selected for pacing in a given heart chamber. The pacing site associated with a particular pacing vector may have a significant effect on the hemodynamic benefit of pacing therapy. As such, pacing vector is one pace control parameter that may be optimized using methods described herein.

A pacing sequence is initiated at block **806** using an initial parameter setting for the test parameter selected at block **804.** In one embodiment, the AV interval is being optimized, and ventricular pacing is delivered at an initial AV interval setting. It is understood that an initial AV interval setting may be selected at block **806** by first measuring an intrinsic AV interval in a patient having intact AV conduction, i.e., no AV block. An initial AV interval may be a default pacing interval, the last programmed AV interval, or a minimum or maximum AV interval to be tested. Alternatively, if the VV interval is selected for optimization, an intrinsic inter-ventricular conduction time may be measured first and paced VV intervals may be iteratively adjusted beginning at a VV interval longer, shorter, or approximately equal to the intrinsic VV conduction time.

An iterative process for adjusting the selected test parameter to at least two different settings is performed. The parameter may be adjusted to different settings in any desired order, e.g., increasing, decreasing, random, etc. For example, during adjustment of AV interval, an initial AV interval may be set to just longer than or approximately equal to a measured intrinsic AV conduction time then iteratively decreased down to a minimum AV interval test setting. During pacing using each pace parameter setting, HS signals are acquired at block **808.** The iterative process advances to the next test parameter setting at block **812** until all test parameter settings have been applied, as determined at block **810,** and HS signals have been recorded for each setting.

HS signals may be acquired for multiple cardiac cycles to enable ensemble averaging or averaging of HS parameter measurements taken from individual cardiac cycles. It is understood that amplification, filtering, rectification, noise cancellation techniques or other signal processing steps may be used for improving the signal-to-noise ratio of the HS signals and these steps may be different for each of the heart sounds being acquired, which may include any or all types of heart sounds.

At least one HS parameter measurement is determined from the recorded HS signals for each test parameter setting at block **814.** The IMD processor or an external processor, e.g., included in a programmer, or a combination of both may perform the HS signal analysis described herein. In one embodiment, S1 and S2 are recorded and HS parameters are measured using the S 1 and S2 signals at block **814.** For example, the amplitude of S 1, the V-S2 interval (where the V event may be a V pace or a sensed R-wave), and the S1-S2 interval are measured. The presence of S3 and/or S4 may additionally be noted, or measurements of these signals may be made for determining related parameters. HS signal parameters are determined for at least two different test parameter settings, e.g., at least two different AV intervals, two or more different VV intervals, or two or more different pacing vectors.

At block **818,** a trend for each HS parameter determined at block **810** as a function of the pace parameter test settings is determined. In one embodiment, a trend for each of the V-S2 interval, S 1 amplitude, and S1-S2 interval is determined. Other embodiments may include determining separation of the M1 and T1 sounds during the S1 signal. Based on the trends of the HS parameter(s) with respect to the varied pace control parameter, an optimal pace parameter setting may be identified automatically by the processor at block **820.** Additionally, or alternatively, the HS trends are reported and displayed at block **822** on an external device such as a programmer or at a remote networked computer.

If the pace parameter being tested is, for example, pacing site or pacing vector when a multipolar electrode is positioned along a heart chamber, such as a quadripolar lead along LV, a pacing site or vector may be selected based on maximizing an HS-based surrogate for ventricular contractility. In one embodiment, the amplitude of S 1 is used as a surrogate for ventricular contractility, and a pacing site or vector associated with a maximum S 1 is identified at block **820** as the optimal pacing vector setting.

Determining the trend of each HS parameter at block **818** may include determining whether the V-S2 interval trend presents a sudden slope change, e.g., from a substantially flat trend to a decreasing trend. An AV interval associated with a sudden change in the V-S2 interval trend may be identified as an optimal AV interval setting. The optimal AV interval may be further identified based on other HS trends, for example, a maximum S1 amplitude and/or a maximum S1-S2 interval.

In some embodiments, an automatically-identified optimal pace parameter setting may also be automatically programmed in the IMD at block **824.** In other embodiments, the clinician or user reviews the reported HS data and recommended pace parameter setting(s) and may accept a recommended setting or select another setting based on the HS data.

HS sensing module, or circuitry, may be operably coupled to the control circuit **80 (****FIG. 8****)** and be configured to receive analog signals from an HS sensor for sensing one or more of these heart sounds. For example, the HS sensing module may include one or more "channels" configured to particularly sense a specific heart sound based on frequency, duration, and timing of the heart sounds. For example, ECG/EGM sensing circuitry may be used by the control circuit **80** to set HS sensing windows used by HS sensing module for sensing the heart sounds. HS sensing module may include one or more sense amplifiers, filters, and rectifiers for optimizing a signal to noise ratio of heart sound signals. Separate and unique amplification and filtering properties may be provided for sensing each of the S 1 through S4 sounds to improve signal quality as needed.

Bioimpedance, or intracardiac impedance, may be measured and used to represent physiological response information. For example, any of the IMDs described herein may measure an intracardiac impedance signal by injecting a current and measuring a voltage between electrodes of an electrode vector configuration (e.g., selected electrodes). For example, the IMD may measure an impedance signal by injecting a current (e.g., a non-pacing threshold current) between a first electrode (e.g., RV electrode) and an electrode located in the RV proximate the tricuspid valve and measuring a voltage between the first and second electrodes. Another vector that may be used is from the LV electrode to the RV electrode. One will recognize that other vector pair configurations may be used for stimulation and measurement. Impedance can be measured between any set of electrodes that encompass the tissue or cardiac chamber of interest. Thus, one can inject current and measure voltage to calculate the impedance on the same two electrodes (a bipolar configuration) or inject current and measure the voltage on two separate pairs of electrodes (e.g., one pair for current injection and one pair for voltage sense), hence, a quadripolar configuration. For a quadripolar electrode configuration, the current injection and voltage sense electrodes may be in line with each other (or closely parallel to) and the voltage sense electrodes may be within the current sense field. For example, if one injected current between the SVC coil electrode and the RV tip electrode, voltage sensing may be between the RV coil electrode and RV ring electrode. In such embodiments, a VfA lead may be used for the LV cardiac therapy or sensing. The impedance vectors can be configured to encompass a particular anatomical area of interest, such as the atrium or ventricles.

The illustrative methods and/or devices described herein may sense one or more electrode vector configurations. Further, multiple impedance vectors may be measured concurrently and/or periodically relative to another. In at least one embodiment, the illustrative methods and/or devices may use impedance waveforms to acquire selection data (e.g., to find applicable fiducial points, to allow extraction of measurements from such waveforms, etc.) for optimizing CRT.

As used herein, the term "impedance signal" is not limited to a raw impedance signal. It should be implied that raw impedance signals may be processed, normalized, and/or filtered (e.g., to remove artifacts, noise, static, electromagnetic interference (EMI), and/or extraneous signals) to provide the impedance signal. Further, the term "impedance signal" may include various mathematical derivatives thereof including real and imaginary portions of the impedance signal, a conductance signal based on the impedance (i.e., the reciprocal or inverse of impedance), etc. In other words, the term "impedance signal" may be understood to include conductance signals, i.e., signals that are the reciprocal of the impedance signal.

In one or more embodiments of the devices described herein, various patient physiological parameters (e.g., intracardiac impedance, heart sounds, cardiac cycle intervals such as R-R interval, etc.) may be determined for use in acquiring selection data to optimize CRT (e.g., set AV and/or VV delay, optimize cardiac contractility, for example, by using and/or measuring impedance first derivative *dZ*/*dt*, select pacing site, select pacing vector, lead placement, or assess pacing capture from both the electrical and mechanical points of view (e.g., electrical capture may not mean mechanical capture, and the heart sounds and impedance may assist in assessing whether the electrical stimulus captures the heart or not by looking at the mechanical information from the heart sounds and impedance), select an effective electrode vector configuration for pacing, etc.). For example, intracardiac impedance signals between two or more electrodes may be acquired for use in providing such optimization.

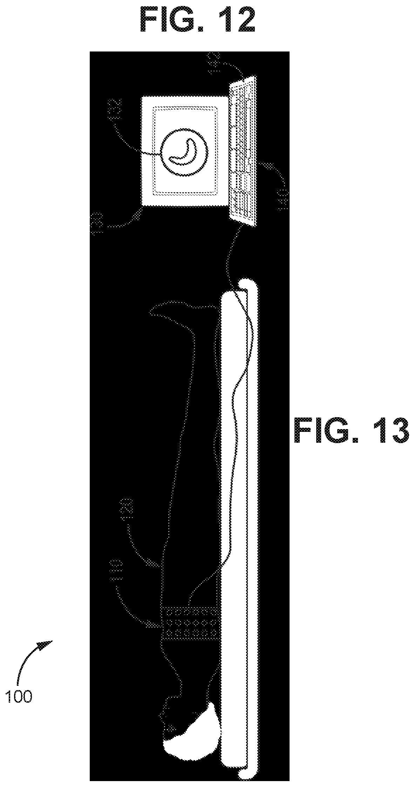

**FIG. 12** shows one example of a non-claimed method **850** for acquiring selection data for one of the device parameter options (e.g., one of the selectable device parameters that may be used to optimize CRT, such as a potential AV delay that may be an optimal parameter). Other examples of using heart sounds to optimize cardiac therapy are described generally in U.S. Patent No. 9,707,399, granted July 18, 2017, entitled "Cardiac resynchronization therapy optimization based on intracardiac impedance and heart sounds".

As shown, pacing therapy is delivered using one of the plurality of device options (block **852)** (e.g., the plurality of device parameter options may be selected, determined and/or calculated AV delays, such as percentages of intrinsic AV delay, for example, 40% of intrinsic AV delay, 50% of intrinsic AV delay, 60% of intrinsic AV delay, 70% of intrinsic AV delay, 80% of intrinsic AV delay, etc.). For the device parameter option used to pace (block **852),** selection data is acquired at each of a plurality of electrode vector configurations (e.g., intracardiac impedance is sensed over a plurality of cardiac cycles, and selection data is extracted using such impedance signal). As indicated by the decision block **854,** if selection data has not been acquired from all desired electrode vector configurations, then the loop of acquiring selection data (e.g., the loop illustrated by blocks **858, 860, 862,** and **864)** is repeated. If selection data has been acquired from all desired electrode vector configurations, then another different device parameter option is used to deliver therapy (block **856)** and the method **850** of **FIG. 12** is repeated (e.g., for the different device parameter option) until selection data has been acquired for all the different device parameter options (e.g., selection data being collected at each of a plurality of electrode vector configurations for each of the different device parameter options).

As shown in the repeated loop of acquiring selection data for each of the desired electrode vector configurations (e.g., blocks **858, 860, 862,** and **864),** one of the plurality of electrode vector configurations is selected for use in acquiring selection data (block **858).** Temporal fiducial points associated with at least a part of a systolic portion of at least one cardiac cycle and/or temporal fiducial points associated with at least a part of a diastolic portion of at least one cardiac cycle for the selected electrode vector configuration are acquired (block **860)** (e.g., such as with use of heart sounds, analysis of impedance signal minimum and maximums, application of algorithms based on physiological parameters such as R-R intervals, etc.). For example, temporal fiducial points associated with the systolic and/or diastolic portions of the cardiac cycle may be acquired, temporal fiducial points associated with one or more defined segments within systolic and/or diastolic portions of the cardiac cycle may be acquired, and/or temporal fiducial points within or associated with one or more points and/or portions of a systolic and/or diastolic portion of the cardiac cycle may be acquired. Yet further, for example, temporal fiducial points associated with just the systolic portion or just the diastolic portion of the cardiac cycle may be acquired, temporal fiducial points associated with one or more defined segments within just the systolic portion or just the diastolic portion of the cardiac cycle may be acquired, and/or temporal fiducial points within or associated with one or more points and/or portions of just the systolic portion or just the diastolic portion of the cardiac cycle may be acquired. In other words, fiducial points may be acquired that are associated with either both the systolic and diastolic portions of the cardiac cycle or associated with just one of such portions of the cardiac cycle. Further, for example, such fiducial points may be representative or indicative of a measurement window and/or time period (e.g., interval, point, etc.) at or during which intracardiac impedance may be measured for use in an analysis as described herein.

In about the same timeframe (e.g., about simultaneously with the acquired fiducial points), an intracardiac impedance signal is acquired at the selected electrode vector configuration (block **862).** With the acquired fiducial points and the acquired intracardiac impedance signal, measurements from the impedance signal are extracted based on the temporal fiducial points (block **864)** (e.g., integral of the impedance signal in a measurement window defined between fiducial points, maximum slope of impedance signal in a measurement window defined between fiducial points, time between the fiducial points, maximum impedance at a fiducial point, etc.). One or more of such measurements may be comparable to desired values for such measurements allowing for a determination of whether the measurement may indicate that the device parameter option may be an effective device parameter for optimizing therapy (e.g., a scoring algorithm may be used to determine if a device parameter option may be an optimal parameter based on whether a plurality of such measurements meet certain criteria or thresholds).

The measurement data for each of the device parameter options (e.g., obtained such as described in **FIG. 12****)** is determined for at least one cardiac cycle. In one or more embodiments, such measurement data is acquired for a plurality of cardiac cycles. The cardiac cycles during which measurement data is acquired may be any suitable cardiac cycle. In one or more embodiments, the selected cardiac cycles during which measurement data is acquired is based on the respiratory cycle. In at least one embodiment, the measurement data is acquired during cardiac cycles occurring at the end of a respiratory cycle (e.g., proximate the end of expiration).

**FIG. 13** depicts an illustrative system **100** including electrode apparatus **110,** display apparatus **130,** and computing apparatus **140.** The electrode apparatus **110** as shown includes a plurality of electrodes incorporated, or included, within a band wrapped around the chest, or torso, of a patient **120.** The electrode apparatus **110** is operatively coupled to the computing apparatus **140** (e.g., through one or wired electrical connections, wirelessly, etc.) to provide electrical signals from each of the electrodes to the computing apparatus **140** for analysis, evaluation, etc. Illustrative electrode apparatus may be described in U.S. Patent No. 9,320,446 entitled "Bioelectric Sensor Device and Methods" and issued on April 26, 2016.

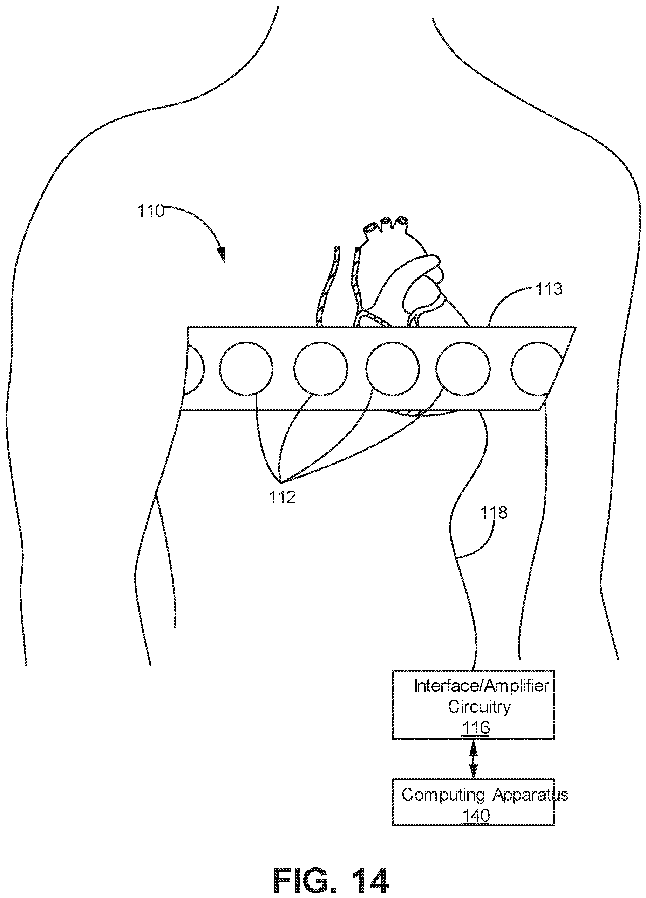

Further, illustrative electrode apparatus **110** will be described in more detail in reference to **FIGS. 14-15****.**

Although not described herein, the illustrative system **100** may further include imaging apparatus. The imaging apparatus may be any type of imaging apparatus configured to image, or provide images of, at least a portion of the patient in a noninvasive manner. For example, the imaging apparatus may not use any components or parts that may be located within the patient to provide images of the patient except noninvasive tools such as contrast solution. It is to be understood that the illustrative systems, methods, and interfaces described herein may further use imaging apparatus to provide noninvasive assistance to a user (e.g., a physician) to calibrate and/or deliver a VfA pacing therapy, to locate and position a device to deliver VfA cardiac pacing therapy, and/or to locate or select a pacing electrode or pacing vector proximate the patient's heart for ventricle from atrium pacing therapy in conjunction with the evaluation of ventricle from atrium pacing therapy.

For example, the illustrative systems and interfaces may provide image-guided navigation that may be used to navigate leads including leadless devices, electrodes, leadless electrodes, wireless electrodes, catheters, etc., within the patient's body while also providing noninvasive cardiac therapy evaluation including determining whether a ventricle from atrium (VfA) paced setting is optimal or determining whether one or more selected parameters are optimal, such as selected location information (e.g., location information for the electrodes to target a particular location in the left ventricle). Illustrative systems and methods that use imaging apparatus and/or electrode apparatus may be described in U.S. Patent Publication No. 2014/0371832 filed on June 12, 2013, and entitled "Implantable Electrode Location Selection," U.S. Patent Publication No. 2014/0371833 filed on June 12, 2013, and entitled "Implantable Electrode Location Selection," U.S. Patent Publication No. 2014/0323892 filed on March 27, 2014 and entitled "Systems, Methods, and Interfaces for Identifying Effective Electrodes," U.S. Patent Publication No. 2014/0323882 filed on March 27, 2014 and entitled "Systems, Methods, and Interfaces for Identifying Optical-Electrical Vectors".

Illustrative imaging apparatus may be configured to acquire x-ray images and/or any other alternative imaging modality. For example, the imaging apparatus may be configured to acquire images, or image data, using isocentric fluoroscopy, bi-plane fluoroscopy, ultrasound, computed tomography (CT), multi-slice computed tomography (MSCT), magnetic resonance imaging (MRI), high frequency ultrasound (HIFU), optical coherence tomography (OCT), intravascular ultrasound (IVUS), two-dimensional (2D) ultrasound, three dimensional (3D) ultrasound, four-dimensional (4D) ultrasound, intraoperative CT, intraoperative MRI, etc. Further, it is to be understood that the imaging apparatus may be configured to acquire a plurality of consecutive images (e.g., continuously) to provide video frame data. In other words, a plurality of images taken over time using the imaging apparatus may provide video frame, or motion picture, data. Additionally, the images may also be obtained and displayed in two, three, or four dimensions. In more advanced forms, four-dimensional surface rendering of the heart or other regions of the body may also be achieved by incorporating heart data or other soft tissue data from a map or from pre-operative image data acquired by MRI, CT, or echocardiography modalities. Image datasets from hybrid modalities, such as positron emission tomography (PET) combined with CT, or single photon emission computer tomography (SPECT) combined with CT, could also provide functional image data superimposed onto anatomical data, e.g., to be used to navigate treatment apparatus proximate target locations (e.g., such as locations within the left ventricle, including a selected location within the high posterior basal and/or septal area of the left ventricular cavity) within the heart or other areas of interest.

Systems and/or imaging apparatus that may be used in conjunction with the illustrative systems and method described herein are described in U.S. Pat. App. Pub. No. 2005/0008210 to Evron et al. published on January 13, 2005, U.S. Pat. App. Pub. No. 2006/0074285 to Zarkh et al. published on April 6, 2006, U.S. Pat. App. Pub. No. 2011/0112398 to Zarkh et al. published on May 12, 2011, U.S. Pat. App. Pub. No. 2013/0116739 to Brada et al. published on May 9, 2013, U.S. Pat. No. 6,980,675 to Evron et al. issued on December 27, 2005, U.S. Pat. No. 7,286,866 to Okerlund et al. issued on October 23, 2007, U.S. Pat. No. 7,308,297 to Reddy et al. issued on December 11, 2011, U.S. Pat. No. 7,308,299 to Burrell et al. issued on December 11, 2011, U.S. Pat. No. 7,321,677 to Evron et al. issued on January 22, 2008, U.S. Pat. No. 7,346,381 to Okerlund et al. issued on March 18, 2008, U.S. Pat. No. 7,454,248 to Burrell et al. issued on November 18, 2008, U.S. Pat. No. 7,499,743 to Vass et al. issued on March 3, 2009, U.S. Pat. No. 7,565,190 to Okerlund et al. issued on July 21, 2009, U.S. Pat. No. 7,587,074 to Zarkh et al. issued on September 8, 2009, U.S. Pat. No. 7,599,730 to Hunter et al. issued on October 6, 2009, U.S. Pat. No. 7,613,500 to Vass et al. issued on November 3, 2009, U.S. Pat. No. 7,742,629 to Zarkh et al. issued on June 22, 2010, U.S. Pat. No. 7,747,047 to Okerlund et al. issued on June 29, 2010, U.S. Pat. No. 7,778,685 to Evron et al. issued on August 17, 2010, U.S. Pat. No. 7,778,686 to Vass et al. issued on August 17, 2010, U.S. Pat. No. 7,813,785 to Okerlund et al. issued on October 12, 2010, U.S. Pat. No. 7,996,063 to Vass et al. issued on August 9, 2011, U.S. Pat. No. 8,060,185 to Hunter et al. issued on November 15, 2011, and U.S. Pat. No. 8,401,616 to Verard et al. issued on March 19, 2013.

The display apparatus **130** and the computing apparatus **140** may be configured to display and analyze data such as, e.g., electrical signals (e.g., electrocardiogram data), cardiac information representative of one or more of mechanical cardiac functionality and electrical cardiac functionality (e.g., mechanical cardiac functionality only, electrical cardiac functionality only, or both mechanical cardiac functionality and electrical cardiac functionality), etc. Cardiac information may include, e.g., electrical heterogeneity information or electrical dyssynchrony information, surrogate electrical activation information or data, etc. that is generated using electrical signals gathered, sensed, or collected, using the electrode apparatus **110.** In at least one embodiment, the computing apparatus **140** may be a server, a personal computer, or a tablet computer. The computing apparatus **140** may be configured to receive input from input apparatus **142** and transmit output to the display apparatus **130.** Further, the computing apparatus **140** may include data storage that may allow for access to processing programs or routines and/or one or more other types of data, e.g., for calibrating and/or delivering pacing therapy for driving a graphical user interface configured to noninvasively assist a user in targeting placement of a pacing device, and/or for evaluating pacing therapy at that location (e.g., the location of an implantable electrode used for pacing, the location of pacing therapy delivered by a particular pacing vector, etc.).

The computing apparatus **140** may be operatively coupled to the input apparatus **142** and the display apparatus **130** to, e.g., transmit data to and from each of the input apparatus **142** and the display apparatus **130.** For example, the computing apparatus **140** may be electrically coupled to each of the input apparatus **142** and the display apparatus **130** using, e.g., analog electrical connections, digital electrical connections, wireless connections, bus-based connections, network-based connections, internet-based connections, etc. As described further herein, a user may provide input to the input apparatus **142** to manipulate, or modify, one or more graphical depictions displayed on the display apparatus **130** and to view and/or select one or more pieces of information related to the cardiac therapy.

Although as depicted the input apparatus **142** is a keyboard, it is to be understood that the input apparatus **142** may include any apparatus capable of providing input to the computing apparatus **140** for performing the functionality, methods, and/or logic described herein. For example, the input apparatus **142** may include a mouse, a trackball, a touchscreen (e.g., capacitive touchscreen, a resistive touchscreen, a multi-touch touchscreen, etc.), etc. Likewise, the display apparatus **130** may include any apparatus capable of displaying information to a user, such as a graphical user interface **132** including cardiac information, textual instructions, graphical depictions of electrical activation information, graphical depictions of anatomy of a human heart, images or graphical depictions of the patient's heart, graphical depictions of a leadless pacing device used to calibrate and/or deliver pacing therapy, graphical depictions of a leadless pacing device being positioned or placed to provide VfA pacing therapy, graphical depictions of locations of one or more electrodes, graphical depictions of a human torso, images or graphical depictions of the patient's torso, graphical depictions or actual images of implanted electrodes and/or leads, etc. Further, the display apparatus **130** may include a liquid crystal display, an organic light-emitting diode screen, a touchscreen, a cathode ray tube display, etc.

The processing programs or routines stored and/or executed by the computing apparatus **140** may include programs or routines for computational mathematics, matrix mathematics, dispersion determinations (e.g., standard deviations, variances, ranges, interquartile ranges, mean absolute differences, average absolute deviations, etc.), filtering algorithms, maximum value determinations, minimum value determinations, threshold determinations, moving windowing algorithms, decomposition algorithms, compression algorithms (e.g., data compression algorithms), calibration algorithms, image construction algorithms, signal processing algorithms (e.g., various filtering algorithms, Fourier transforms, fast Fourier transforms, etc.), standardization algorithms, comparison algorithms, vector mathematics, or any other processing required to implement one or more illustrative methods and/or processes described herein. Data stored and/or used by the computing apparatus **140** may include, for example, electrical signal/waveform data from the electrode apparatus **110,** dispersions signals, windowed dispersions signals, parts or portions of various signals, electrical activation times from the electrode apparatus **110,** graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein (e.g., electrical signals, cardiac information, etc.), or any other data that may be necessary for carrying out the one and/or more processes or methods described herein.

In one or more embodiments, the illustrative systems, methods, and interfaces may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform the functionality described herein and generate desired output information. The output information may be applied as input to one or more other devices and/or methods as described herein or as would be applied in a known fashion.

The one or more programs used to implement the systems, methods, and/or interfaces described herein may be provided using any programmable language, e.g., a high-level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the illustrative systems, methods, and/or interfaces may be implemented using a computer readable storage medium, configured with a computer program, where the storage medium so configured causes the computer to operate in a specific and predefined manner to perform functions described herein. Further, in at least one embodiment, the illustrative systems, methods, and/or interfaces may be described as being implemented by logic (e.g., object code) encoded in one or more non-transitory media that includes code for execution and, when executed by a processor, is operable to perform operations such as the methods, processes, and/or functionality described herein.

The computing apparatus **140** may be, for example, any fixed or mobile computer system (e.g., a controller, a microcontroller, a personal computer, minicomputer, tablet computer, etc.) and may be generally described as including processing circuitry. The exact configuration of the computing apparatus **140** is not limiting, and essentially any device capable of providing suitable computing capabilities and control capabilities (e.g., graphics processing, etc.) may be used. As described herein, a digital file may be any medium (e.g., volatile or non-volatile memory, a CD-ROM, a punch card, magnetic recordable medium such as a disk or tape, etc.) containing digital bits (e.g., encoded in binary, trinary, etc.) that may be readable and/or writeable by computing apparatus **140** described herein. Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes, or programs (e.g., the functionality provided by such systems, processes, or programs) described herein.

Electrical activation times of the patient's heart may be useful to evaluate a patient's cardiac condition and/or to calibrate, deliver, or evaluate ventricle from atrium (VfA) cardiac therapy to be or being delivered to a patient. Surrogate electrical activation information or data of one or more regions of a patient's heart may be sensed, or determined, using electrode apparatus **110** as shown in **FIGS. 13-15****.** The illustrative electrode apparatus **110** may be configured to measure body-surface potentials of a patient **120** and, more particularly, torso-surface potentials of a patient **120.**

As shown in **FIG. 14****,** the illustrative electrode apparatus **110** may include a set, or array, of electrodes **112,** a strap **113,** and interface/amplifier circuitry **116.** In at least one embodiment, a portion of the set of electrodes may be used wherein the portion corresponds to a particular location on the patient's heart. The electrodes **112** may be attached, or coupled, to the strap **113,** and the strap **113** may be configured to be wrapped around the torso of a patient **120** such that the electrodes **112** surround the patient's heart. As further illustrated, the electrodes **112** may be positioned around the circumference of a patient **120,** including the posterior, lateral, posterolateral, anterolateral, and anterior locations of the torso of a patient **120.**

Further, the electrodes **112** may be electrically connected to interface/amplifier circuitry **116** via wired connection **118.** The interface/amplifier circuitry **116** may be configured to amplify the signals from the electrodes **112** and provide the signals to the computing apparatus **140.** Other illustrative systems may use a wireless connection to transmit the signals sensed by electrodes **112** to the interface/amplifier circuitry **116** and, in turn, the computing apparatus **140,** e.g., as channels of data. For example, the interface/amplifier circuitry **116** may be electrically coupled to each of the computing apparatus **140** and the display apparatus **130** using, e.g., analog electrical connections, digital electrical connections, wireless connections, bus-based connections, network-based connections, internet-based connections, etc.

Although in the example of **FIG. 14** the electrode apparatus **110** includes a strap **113,** in other examples any of a variety of mechanisms, e.g., tape or adhesives, may be employed to aid in the spacing and placement of electrodes **112.** In some examples, the strap **113** may include an elastic band, strip of tape, or cloth. In other examples, the electrodes **112** may be placed individually on the torso of a patient **120.** Further, in other examples, electrodes **112** (e.g., arranged in an array) may be part of, or located within, patches, vests, and/or other manners of securing the electrodes **112** to the torso of the patient **120.**

The electrodes **112** may be configured to surround the heart of the patient **120** and record, or sens, the electrical signals associated with the depolarization and repolarization of the heart after the signals have propagated through the torso of a patient **120.** Each of the electrodes **112** may be used in a unipolar configuration to sense the torso-surface potentials that reflect the cardiac signals. The interface/amplifier circuitry **116** may also be coupled to a return or indifferent electrode (not shown) that may be used in combination with each electrode **112** for unipolar sensing. In some examples, there may be about 12 to about 50 electrodes **112** spatially distributed around the torso of the patient. Other configurations may have more or fewer electrodes **112.**

The computing apparatus **140** may record and analyze the electrical activity (e.g., torso-surface potential signals) sensed by electrodes **112** and amplified/conditioned by the interface/amplifier circuitry **116.** The computing apparatus **140** may be configured to analyze the signals from the electrodes **112** to provide as anterior and posterior electrode signals and surrogate cardiac electrical activation times, e.g., representative of actual, or local, electrical activation times of one or more regions of the patient's heart as will be further described herein. The computing apparatus **140** may be configured to analyze the signals from the electrodes **112** to provide as anterior-septal electrode signals and surrogate cardiac electrical activation times, e.g., representative of actual, or local, electrical activation times of one or more anterior-septal regions of the patient's heart, as will be further described herein, e.g., for use in calibrating, delivering, and/or evaluating VfA pacing therapy. Further, the electrical signals measured at the left anterior surface location of a patient's torso may be representative, or surrogates, of electrical signals of the left anterior left ventricle region of the patient's heart, electrical signals measured at the left lateral surface location of a patient's torso may be representative, or surrogates, of electrical signals of the left lateral left ventricle region of the patient's heart, electrical signals measured at the left posterolateral surface location of a patient's torso may be representative, or surrogates, of electrical signals of the posterolateral left ventricle region of the patient's heart, and electrical signals measured at the posterior surface location of a patient's torso may be representative, or surrogates, of electrical signals of the posterior left ventricle region of the patient's heart. In one or more embodiments, measurement of activation times can be performed by measuring the period of time between an onset of cardiac depolarization (e.g., onset of QRS complex) and an appropriate fiducial point such as, e.g., a peak value, a minimum value, a minimum slope, a maximum slope, a zero crossing, a threshold crossing, etc.

Additionally, the computing apparatus **140** may be configured to provide graphical user interfaces depicting the surrogate electrical activation times obtained using the electrode apparatus **110.** Illustrative systems, methods, and/or interfaces may noninvasively use the electrical information collected using the electrode apparatus **110** to evaluate a patient's cardiac condition and/or to calibrate, deliver, or evaluate VfA pacing therapy to be or being delivered to the patient.

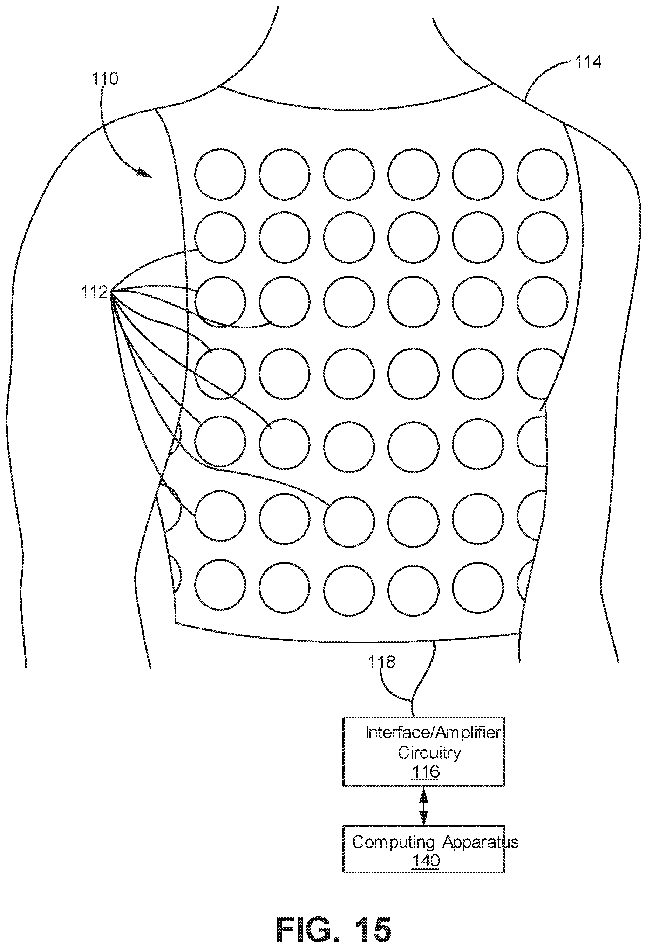

**FIG. 15** illustrates another illustrative electrode apparatus **110** that includes a plurality of electrodes **112** configured to surround the heart of the patient **120** and record, or sens, the electrical signals associated with the depolarization and repolarization of the heart after the signals have propagated through the torso of the patient **120.** The electrode apparatus **110** may include a vest **114** upon which the plurality of electrodes **112** may be attached, or to which the electrodes **112** may be coupled. In at least one embodiment, the plurality, or array, of electrodes **112** may be used to collect electrical information such as, e.g., surrogate electrical activation times.

Similar to the electrode apparatus **110** of **FIG. 14****,** the electrode apparatus **110** of **FIG. 13** may include interface/amplifier circuitry **116** electrically coupled to each of the electrodes **112** through a wired connection **118** and be configured to transmit signals from the electrodes **112** to computing apparatus **140.** As illustrated, the electrodes **112** may be distributed over the torso of a patient **120,** including, for example, the anterior, lateral, posterolateral, anterolateral, and posterior surfaces of the torso of the patient **120.**

The vest **114** may be formed of fabric with the electrodes **112** attached to the fabric. The vest **114** may be configured to maintain the position and spacing of electrodes **112** on the torso of the patient **120.** Further, the vest **114** may be marked to assist in determining the location of the electrodes **112** on the surface of the torso of the patient **120.** In one or more embodiments, the vest **114** may include about 17 or more anterior electrodes positionable proximate the anterior torso of the patient, and about 39 or more posterior electrodes positionable proximate the anterior torso of the patient. In some examples, there may be about 25 electrodes **112** to about 256 electrodes **112** distributed around the torso of the patient **120**, though other configurations may have more or fewer electrodes **112**.

As described herein, the electrode apparatus **110** may be configured to measure electrical information (e.g., electrical signals) representing different regions of a patient's heart. For example, activation times of different regions of a patient's heart can be approximated from surface electrocardiogram (ECG) activation times measured using surface electrodes in proximity to surface areas corresponding to the different regions of the patient's heart. In at least one example, activation times of the anterior-septal region of a patient's heart can be approximated from surface ECG activation times measured using surface electrodes in proximity to surface areas corresponding to the anterior-septal region of the patient's heart. That is, a portion of the set of electrodes **112**, and not the entire set, can be used to generate activation times corresponding to a particular location of the patient's heart that the portion of the set of electrodes corresponds to.

The illustrative systems and interfaces may be used to provide noninvasive assistance to a user in the evaluation of a patient's cardiac health or status, and/or the evaluation of cardiac therapy such as ventricle from atrium (VfA) pacing therapy by use of the electrode apparatus **110** (e.g., cardiac therapy being presently-delivered to a patient during implantation or after implantation). Further, the illustrative systems, methods, and interfaces may be used to assist a user in the configuration, or calibration, of the cardiac therapy, such as VfA pacing therapy, to be or being delivered to a patient.

VfA pacing can be described as providing a synchronized homogeneous activation of ventricles of the heart. As an example, patients with atrial-ventricular (AV) block or prolonged AV timings that can lead to heart failure who have otherwise intact (e.g., normal) QRS can benefit from VfA pacing therapy. In addition, as an example, VfA pacing may provide beneficial activation for heart failure patients with intrinsic ventricular conduction disorders. Further, proper placement of VfA pacing can provide optimal activation of the ventricles for such patients. Further, left ventricular (LV) resynchronization for heart failure patients with left bundle branch block (LBBB) may find that VfA pacing enables easier access to left ventricular endocardium without exposing the leadless device or lead to the endocardial blood pool. At the same time, in that example, this can help engage part of the conduction system to potentially correct LBBB and effectively resynchronize the patient.

Electrical activity may be sensed using a plurality of external electrodes, such as electrodes **112** of **FIGS. 13-15**. The electrical activity can be sensed by a plurality of electrodes during VfA pacing therapy or in the absence of VfA pacing therapy. The sensed electrical activity can be used to evaluate VfA pacing therapy to a patient. The electrical activity sensed using the ECG belt described can be used to evaluate at least one paced setting of the VfA pacing therapy on the heart. As an example, a paced setting can be any one parameter or a combination of parameters including, but not limited to, electrode position, pacing polarity, pacing output, pacing pulse width, timing at which VfA pacing is delivered relative to atrial (A) timing, pacing rate, etc. Further, as an example, the location of the leadless device or a pacing lead can include a location in the left ventricle, accessed through the right atrium within, or in close proximity to, the high posterior basal and/or septal (HPBS) area of the left ventricular cavity. Moreover, pacing in, or in close proximity to, the HPBS area can be selective (e.g., involving stimulation of a particular area of the HPBS alone) or non-selective (e.g., combined pacing at the location of the HPBS and other atrial and/or ventricular septum areas).

Further, body-surface isochronal maps of ventricular activation can be constructed using the sensed electrical activity during VfA pacing therapy or in the absence of VfA pacing therapy. The sensed electrical activity and/or the map of ventricular activation can be used to generate electrical heterogeneity information (EHI). The electrical heterogeneity information can include determining metrics of electrical heterogeneity. The metrics of electrical heterogeneity can include a metric of standard deviation of activation times (SDAT) of electrodes on a left side of a torso of the patient and/or a metric of mean left ventricular activation time (LVAT) of electrodes on the left side of the torso of the patient. A metric of LVAT may be determined from electrodes on both the anterior and posterior surfaces, which are more proximal to the left ventricle. The metrics of electrical heterogeneity information can include a metric of mean right ventricular activation time (RVAT) of electrodes on the right side of the torso of the patient. A metric of RVAT may be determined from electrodes on both the anterior and posterior surfaces which are more proximal to the right ventricle. The metrics of electrical heterogeneity can include a metric of mean total activation time (mTAT) taken from a plurality of electrode signals from both sides of the torso of the patient, or it may include other metrics (e.g., standard deviation, interquartile deviations, a difference between a latest activation time and earliest activation time) reflecting a range or dispersion of activation times on a plurality of electrodes located on the right side of the patient torso or left side of the patient torso, or combining both right and left sides of the patient torso. The metrics of electrical heterogeneity information can include a metric of anterior-septal activation times (ASAT) of electrodes on the torso in close proximity to the anterior-septal portion of the heart.

Electrical heterogeneity information (EHI) may be generated during delivery of VfA pacing therapy at one or more VfA paced settings. The electrical heterogeneity information can be generated using metrics of electrical heterogeneity. As an example, the metrics of electrical heterogeneity can include one or more of an SDAT, an LVAT, an RVAT, an mTAT, and an ASAT. In at least one embodiment, only ASAT may be determined and further used, and/or ASAT may be more heavily weighted than other values.

One or more paced settings associated with the VfA pacing therapy may be evaluated. A paced setting can include a plurality of pacing parameters. The plurality of pacing parameters can be optimal if the patient's cardiac condition improves, if the VfA pacing therapy is effectively capturing a desired portion of the left ventricle (e.g., the high posterior basal and/or septal area), and/or if a metric of electrical heterogeneity improves by a certain threshold compared to a baseline rhythm or therapy. In at least one embodiment, the determination of whether the paced setting is optimal can be based on at least one metric of electrical heterogeneity generated from electrical activity during VfA pacing (and also, in some embodiments, during native conduction, or in the absence of VfA pacing). The at least one metric can include one or more of an SDAT, an LVAT, an RVAT, an mTAT, and an ASAT.

Further, the plurality of pacing parameters can be optimal if a metric of electrical heterogeneity is greater than or less than a particular threshold, and/or if the location of the pacing therapy to excite the left ventricle causes a particular pattern of excitation of the muscle fibers in the heart. In addition, the plurality of pacing parameters can be optimal if a metric of electrical heterogeneity indicates a correction of a left bundle branch block (LBBB), and/or if a metric of electrical heterogeneity indicates a complete engagement of a Purkinje system, etc. As an example, a metric of electrical heterogeneity of an ASAT less than or equal to a threshold (e.g., a threshold of 30 ms) and an LVAT less than or equal to a threshold (e.g., a threshold of 30 ms) can indicate a correction of an LBBB, and thus, the paced setting is optimal. As an example, a metric of electrical heterogeneity of an RVAT less than or equal to a threshold (e.g., a threshold of 30 ms), an ASAT less than or equal to a threshold (e.g., a threshold of 30 ms), and an LVAT less than or equal to a threshold (e.g., a threshold of 30 ms) can indicate a complete engagement of the Purkinje system, and thus the paced setting is may be optimal.

The paced setting can be determined to be optimal in response to the VfA pacing therapy using the paced setting being acceptable, being beneficial, being indicative of complete engagement of patient's native cardiac conduction system, being indicative of correction of a ventricular conduction disorder (e.g., left bundle branch block), etc. A paced setting can include one or more of a pacing electrode position (including one or more of a depth, an angle, an amount of turn for a screw-based fixation mechanism, etc.), a voltage, a pulse width, an intensity, a pacing polarity, a pacing vector, a pacing waveform, a timing of the pacing delivered relative to an intrinsic or paced atrial event or relative to the intrinsic His bundle potential, and/or a pacing location, etc. A pacing vector can include any two or more pacing electrodes such as, e.g., a tip electrode to a can electrode, a tip electrode to a ring electrode etc., that are used to deliver the VfA pacing therapy, etc. The pacing location can refer to the location of any of the one or more pacing electrodes that are positioned using a lead, a leadless device, and/or any device or apparatus configured to deliver VfA.

A paced setting for VfA pacing therapy may be adjusted. In at least one embodiment, the paced setting can be adjusted in response to the paced setting being not optimal. In at least one embodiment, the paced setting can be adjusted in response to the paced setting being within an optimal range but in order to determine whether the paced setting can be at a position within the optimal range that is more beneficial, more useful, more functional, etc., for the VfA pacing therapy. The paced setting could be adjusted to find the most optimal metric of electrical heterogeneity.

In one or more embodiments, a determination of whether the paced setting is optimal can be based on a particular metric of electrical heterogeneity using an ECG belt. In at least one example, the paced setting can be adjusted at intervals that correlate with a change in the metric of electrical heterogeneity until the metric of electrical heterogeneity is at or proximate a particular metric value. For instance, the adjusting of the paced setting can cause the metric of electrical heterogeneity to approach a particular threshold metric of electrical heterogeneity and, as the metric approaches the particular threshold, the rate at which the paced setting is adjusted can be slowed down. Put another way, as the metric of electrical heterogeneity is further from the particular threshold metric, the paced setting can be adjusted more quickly and as the metric of electrical heterogeneity gets closer to the particular threshold metric, the paced setting can be adjusted more slowly until the metric of electrical heterogeneity is at the particular threshold metric.

Various techniques for utilizing an electrode apparatus having a plurality of external electrodes to sense electrical activity from tissue of a patient that may be used with the devices, systems, and methods described herein are disclosed in U.S. Patent Application Serial No. 15/934,517, filed 23 March 2018, entitled "Evaluation of Ventricle from Atrium Pacing Therapy".

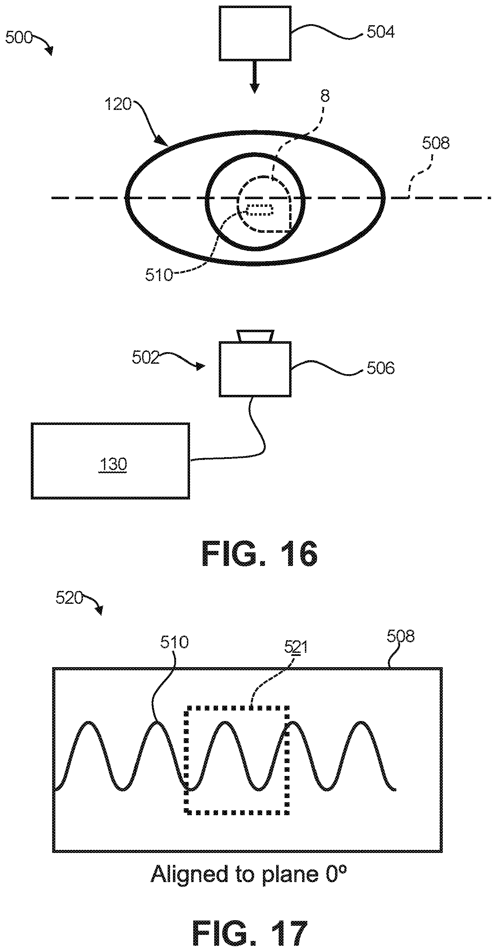

**FIG. 16** shows an overhead view of an environment **500** including the patient **120** and an imaging device **502** having an imaging source **504** and an imaging receiver **506**. In some embodiments, the imaging device **502** may be an x-ray machine having an x-ray source and an x-ray receiver. An imageable member **510** may be viewed using the imaging device **502** while being implanted in the patient's heart **8**. The imaging device **502** may provide 2D imaging information, for example, aligned to a plane **508**. The plane **508** may have a normal that is aligned, or parallel to, the direction of imaging between the imaging source **504** and the imaging receiver **506**. Various imaging techniques and imageable members that may be used with the techniques of the present disclosure may be found, for example, in U.S. Patent No. 10,004,467, issued June 26, 2018, entitled "Guidance system for localization and cannulation of the coronary sinus".

In some embodiments, the imageable member **510** may be part of, or include, one or more electrodes of an implantable medical device. In one example, the imageable member **510** may be part of, or include a tissue-piercing electrode implantable to deliver cardiac therapy to or sense electrical activity of the left ventricle in the basal region, septal region, or basal-septal region of the left ventricular myocardium of a patient's heart. In another example, the imageable member **510** may be part of, or include, a right atrial electrode or any other suitable electrode that is implantable in the patient's heart **8**.

In some embodiments, one or more electrodes may be coupled to a housing of the implantable medical device leadlessly or using a lead. In one example, in an intracardiac device, various components may be located within an implantable housing, such as a therapy delivery circuit, a sensing circuit, and a controller, and a tissue-piercing electrode, which may be formed of an imageable material, may be leadlessly coupled to the housing. In another example, which may be an intracardiac device, various components may be located within an implantable housing, such as a therapy delivery circuit, a sensing circuit, and a controller, and a tissue-piercing electrode may be coupled to a lead that extends from the housing to a distal end region. In particular, the tissue-piercing electrode may be coupled to the distal end region of the lead. A right atrial electrode may also be coupled to the distal end region of the lead.

In general, imageable member **510** may be analyzed and used to provide additional information beyond the information shown in the image. For example, the imageable member **510** may be configured, when viewed using the imaging device **502** to provide 2D image information, to provide orientation information in a third dimension orthogonal to the two dimensions of the two-dimensional imaging (e.g., orientation information in the z-axis based on imaging information in the xy-plane). In other words, the 3D orientation information may be generated from 2D image information.

In some embodiments, the imaging device **502** may be operably coupled to a display apparatus, such as display apparatus **130**, to show the imaging information to a user. In some embodiments, the imaging device **502** may be operably coupled to a computing apparatus (not shown), such as computing apparatus **140** (**FIG. 13**), to further process the imaging information. For example, the computing apparatus may be used to determine a 3D orientation, and a representation of the 3D orientation may be shown to the user.

**FIGS. 17-23** show images of various orientations of the imageable member **510** of an implantable medical device as seen in 2D imaging information in the plane **508 (****FIG. 16****)** from the imaging device **502** (**FIG. 16**)**.** The imageable member **510** is at least partially made of an imageable material. In the illustrated embodiment, the imageable member **510** has a helical shape and a right-handed winding (e.g., going from left to right). In some embodiments, the imageable member **510** as shown may be used as a tissue-piercing electrode.

**FIG. 17** shows an image **520** of the imageable member **510** at 0 degrees relative to the plane **508**. In other words, the imageable member **510** is aligned to the plane **508**. As can be seen in section **521** of the image **520**, the imageable member **510** looks like a sine wave.

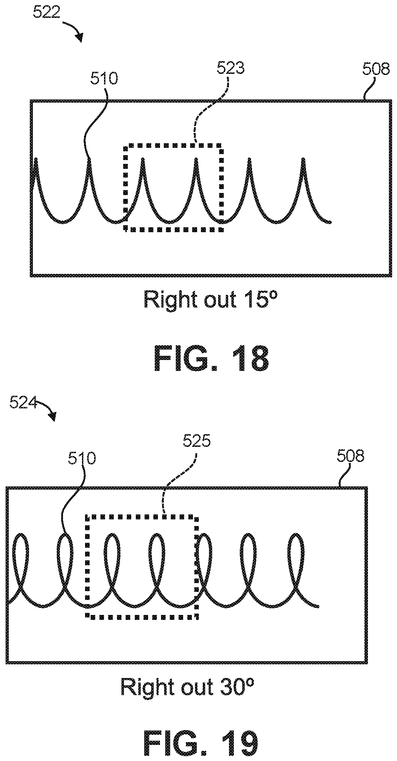

**FIG. 18** shows an image **522** of the imageable member **510** at +15 degrees relative to the plane **508**. In other words, the imageable member **510** is angled away from the imaging receiver of the imaging device **502.** As can be seen in section **523** of the image **522**, the imageable member **510** looks like a repeating U-shape.

**FIG. 19** shows an image **524** of the imageable member **510** at +30 degrees relative to the plane **508.** In other words, the imageable member **510** is angled even further away from the imaging receiver of the imaging device **502** than shown in **FIG. 18**. As can be seen in section **525** of the image **524**, the imageable member **510** looks like a repeating U-shape with small loops at the end, or top, of each U.

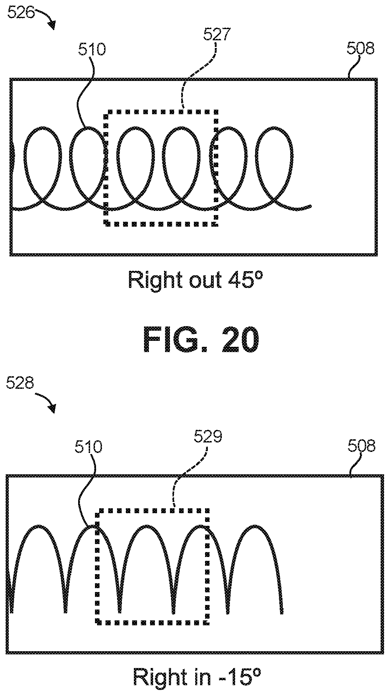

**FIG. 20** shows an image **526** of the imageable member **510** at +45 degrees relative to the plane **508**. In other words, the imageable member **510** is angled even further away from the imaging receiver of the imaging device **502** than shown in **FIGS. 18-19**. As can be seen in section **527** of the image **526**, the imageable member **510** looks like a repeating U-shape with large loops at the end, or top, of each U.

**FIG. 21** shows an image **528** of the imageable member **510** at -15 degrees relative to the plane **508** (e.g., the opposite from **FIG. 18**). In other words, the imageable member **510** is angled toward the imaging receiver of the imaging device **502**. As can be seen in section **529** of the image **528**, the imageable member **510** looks like a repeating lowercase N-shape (e.g., an arch).

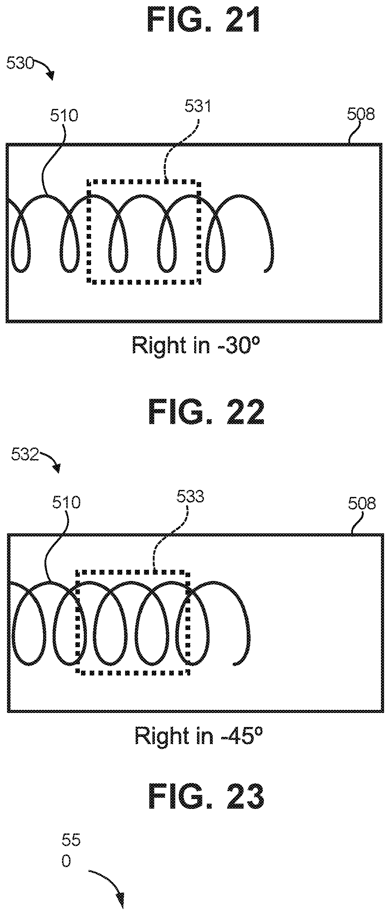

**FIG. 22** shows an image **530** of the imageable member **510** at -30 degrees relative to the plane **508** (e.g., the opposite angle from **FIG. 19**). In other words, the imageable member **510** is angled even further toward the imaging receiver of the imaging device **502** than shown in **FIG. 21**. As can be seen in section **531** of the image **530**, the imageable member **510** looks like a repeating lowercase N-shape with small loops at the end, or bottom, of each N.

**FIG. 23** shows an image **532** of the imageable member **510** at -45 degrees relative to the plane **508** (e.g., the opposite angle from **FIG. 20**). In other words, the imageable member **510** is angled even further toward the imaging receiver of the imaging device **502** than shown in **FIGS. 21-22**. As can be seen in section **533** of the image **532**, the imageable member **510** looks like a repeating lowercase N-shape with large loops at the end, or top, of each N.

Knowledge of the shapes of the imageable member **510** visible in the two-dimensional imaging may be used in one or more implantable medical device delivery methods, or implantation methods.

**FIG. 24** shows one example of a non-claimed method **550** of delivering an implantable medical device, such as an implantable medical device described hereinabove. The method **550** may include advancing a medical device **552**. For example, an implantable medical device having a tissue-piercing electrode may be advanced toward the triangle of Koch region of the right atrium through the right atrial endocardium and central fibrous body. The tissue-piercing electrode may be configured to deliver cardiac therapy to or sense electrical activity of the left ventricle in the basal region, septal region, or basal-septal region of the left ventricular myocardium of a patient's heart. In some embodiments, the tissue-piercing electrode may be formed of an imageable material. In some embodiments, the implantable medical device may include an imageable member formed of an imageable material coupled to a housing or a lead of the implantable medical device.

The method **550** may include acquiring image information **554**. For example, image information may be acquired that represents the patient's heart and that represents the imageable material. The image information may be generated using two-dimensional imaging (e.g., x-ray fluoroscopy).

Further, the method **550** may include generating orientation information **556**. For example, orientation information representing the implantable medical device may be generated based on the acquired image information. The orientation of the implantable medical device may be determined from the generated orientation information. In one example, processing circuitry may be used to determine the orientation of the implantable medical device based on the generated orientation information. As used herein, "orientation information" refers to any information derived from the imaging information that may be used to provide information in another dimension that is not shown in the imaging information. For example, orientation information may include the detection of the U-shape or the N-shape of the imageable member (see, e.g., **FIGS. 18-****23**).

The method **550** may also include implanting the implantable medical device **558**. In one example, the implantable medical device may be implanted in the triangle of Koch region of the right atrium of the patient's heart based on the generated orientation information representing the implantable medical device. In another example, the implantable medical device may be implanted from the coronary sinus into the myocardium adjacent to the cardiac conduction system.

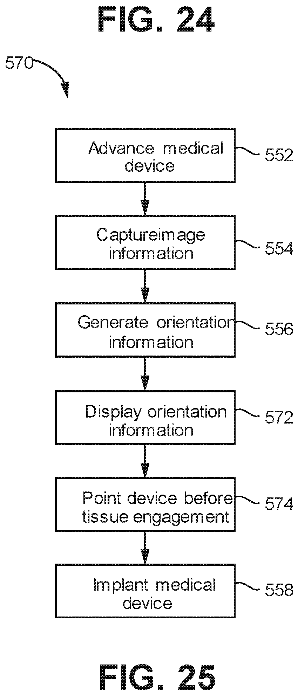

**FIG. 25** shows another example of a non-claimed method **570** of delivering an implantable medical device. Method **570** may also include one or more of advancing the medical device **552**, acquiring image information **554**, generating orientation information **556**, and implanting the medical device **558**.

In addition, method **570** may include displaying orientation information **572**. In one example, the imageable member having imageable material may be displayed on a display device (e.g., display apparatus) and show at least one of a lowercase N-shape (e.g., arch) or a U-shape viewable in the two-dimensional imaging. The lowercase N-shape or U-shape may be used to indicate whether the implantable medical device is pointed toward or away from an imaging device used to acquire the image information.

The method **570** may also include pointing the device before tissue engagement **574**. In some embodiments, once the general implantation site (e.g., triangle of Koch region) has been reached a user may be instructed, or may have been instructed, to point the implantable medical device (e.g., the tissue-piercing electrode end portion) toward the apex of the patient's heart. Pointing the device in this manner may provide an optimal angle to reach the left ventricular myocardium or cardiac conduction system. In particular, the user may point the device before advancing the tissue-piercing electrode into the tissue of the patient's heart (e.g., into the right atrial endocardium and central fibrous body). A predetermined model of the patient's heart may be used to facilitate visualization and guidance to reach the general implantation site and the orientation of the implantable medical device. Any generated orientation information may also be used to facilitate visualization and guidance of the implantable medical device.

Generally, positioning of an electrode within the patient can be based upon image data acquired of the patient and user input and experience. Positioning of the electrode depend on the geometry of the chambers of the heart or vessels, tortuosity of a path through the chambers of the heart or vessels to reach a target location, the diameter of the coronary vessels, or any combination of these.

Using various systems, such as the CardioGuide^{™} implant system sold by Medtronic, Inc., image data can be acquired of a patient to identify various portions of the anatomy. For example, 2D image data acquired during venograms can be assimilated or reconstructed into a 2D model of the coronary vasculature of a subject, such as a human patient. The 3D image of the vasculature can include geometric, size, and configuration information regarding various portions of the anatomy, tortuosity of a path to reach a target location, vessel size to reach a coronary vessel, size of the vessel, location of the vessel, etc. In addition to the geometric shape and configuration of the patient, image data can incorporate or include physiological data, such as contraction timing, motion change, and the like. For example, image data can be acquired at a single time to generate a static three-dimensional model. Image data may also be acquired over time, such as about 30 frames per second, to acquire contraction timing data regarding a patient.

A system that can generate images of a patient, such as a vascular system of a patient, using fluoroscopic images may require the injection of a contrast agent into the vasculature of a patient. To inject a contrast agent into a vasculature of a heart of a patient, access to a coronary sinus ostium (herein CSOs) may be obtained. According to various embodiments a catheter can be positioned in or near the CSOs and a contrast agent can be injected to allow a contrast agent to flow through a vasculature around a heart. Images can be acquired of the patient, such as with a fluoroscopic system, during the flow time of the contrast agent.

Prior to the injection of a contrast agent into a patient's vascular system, fluoroscopic images (generally using x-rays) may not provide high contrast view of non-radiopaque portions. For example, non-radiopaque portions can include soft tissues of the heart. A high contrast instrument, such as a radiopaque portion of a catheter, can be viewed with a fluoroscopic imaging system.

A statistical model or model of a statistically "average subject" can be used to assist in identifying a possible or statistically probable location of the CSOs. A user, such as a surgeon, can then move the catheter towards a statistically probable location and orientation of the CSOs to assist in cannulating the CSOs and allowing injection of a contrast agent into the CSOs for flowing through the vascular system of the patient. As discussed herein, various techniques can be used to assist in cannulating the CSOs, such as real time tracking (i.e. viewing) of the relative position of an instrument and the predicted CSOs location, measurement of the instrument location and direction based on radiopaque landmarks and views of the instrument, etc.

Thus, various embodiments of IMPLANTABLE MEDICAL DEVICE DELIVERY FOR CARDIAC THERAPY are disclosed. Although reference is made herein to the accompanying set of drawings that form part of this disclosure, one of at least ordinary skill in the art will appreciate that various adaptations and modifications of the embodiments described herein are within, or do not depart from, the scope of this disclosure. For example, aspects of the embodiments described herein may be combined in a variety of ways with each other. Therefore, it is to be understood that, within the scope of the appended claims, the claimed invention may be practiced other than as explicitly described herein.

It will be understood that each block of the block diagrams and combinations of those blocks can be implemented by means for performing the illustrated function.

(Deleted)

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims may be understood as being modified either by the term "exactly" or "about." Accordingly, unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

The recitation of numerical ranges by endpoints includes all numbers subsumed within that range (e.g., 1 to 5 includes 1, 1.5, 2, 2.75, 3, 3.80, 4, and 5) and any range within that range. Herein, the terms "up to" or "no greater than" a number (e.g., up to 50) includes the number (e.g., 50), and the term "no less than" a number (e.g., no less than 5) includes the number (e.g., 5).

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out at least some functionality.

## Claims

1. An implantable medical device comprising:
one or more electrodes (22, 24, 42) comprising a tissue-piercing electrode (12) implantable from the triangle of Koch region of the right atrium through the right atrial endocardium and central fibrous body to deliver cardiac therapy to or sense electrical activity of the left ventricle in the basal region, septal region, or basal-septal region of the left ventricular myocardium of a patient's heart, and
an imageable member (20) comprising an imageable material coupled to a housing or a lead configured, when viewed using two-dimensional imaging, to provide orientation information in a third dimension orthogonal to the two dimensions;
a therapy delivery circuit (84) operably coupled to the one or more electrodes to deliver cardiac therapy to the patient's heart;
a sensing circuit (86) operably coupled to the one or more electrodes to sense electrical activity of the patient's heart; and
a controller (80) comprising processing circuitry operably coupled to the therapy delivery circuit and the sensing circuit, the controller configured to:
sense electrical activity using the one or more electrodes; and
deliver cardiac therapy based on the sensed electrical activity.

2. The device according to claim 1, wherein the imageable material comprises radiopaque properties visible using fluoroscopy.

3. The device according to any preceding claim, wherein the one or more electrodes comprise a right atrial electrode positionable within the right atrium to deliver cardiac therapy to or sense electrical activity of the right atrium of the patient's heart.

4. The device according to any preceding claim, wherein the tissue-piercing electrode is configured, when viewed using two-dimensional imaging, to provide orientation information in a third dimension orthogonal to the two dimensions of the two-dimensional imaging.

5. The device according to any preceding claim, wherein the tissue-piercing electrode has a helical shape.

6. The device according to any preceding claim, further comprising a housing (420), wherein the therapy delivery circuit, the sensing circuit, and the controller are located within the housing and the tissue-piercing electrode is leadlessly coupled to the housing.

7. The device according to any of claims 1 to 5, further comprising:
a housing (420), wherein the therapy delivery circuit, the sensing circuit, and the controller are located within the housing; and
a lead (410) coupled to and extending from the housing to a distal end region, wherein the tissue-piercing electrode and a right atrial electrode are coupled to the distal end region of the lead.

8. The device according to any of claims 1 to 5, further comprising:
a housing (420), wherein the therapy delivery circuit, the sensing circuit, and the controller are located within the housing;
a first lead (410) coupled to and extending from the housing to a first distal end region, wherein the tissue-piercing electrode is coupled to the first distal end region of the first lead; and
a second lead (412) coupled to and extending from the housing to a second distal end region, wherein a right atrial electrode is coupled to the second distal end region of the second lead.

9. The device according to any of claims 1 to 5, further comprising:
a housing (420), wherein the therapy delivery circuit, the sensing circuit, and the controller are located within the housing;
a first lead (410) coupled to and extending from the housing to a first distal end region wherein the tissue-piercing electrode is coupled to the first distal end region of the first lead;
a second lead (412) coupled to and extending from the housing to a second distal end region, wherein a right atrial electrode is coupled to the second distal end region of the second lead; and
a third lead (414) coupled to and extending from the housing to a third distal end region, wherein the one or more electrodes further comprise a right ventricular electrode to deliver cardiac therapy to or sense electrical activity of the right ventricle of the patient's heart coupled to the third distal end region of the third lead.

10. The device according to any of claims 1 to 9, wherein the imageable member is part of, or includes the tissue-piercing electrode.

## Patentansprüche

1. Implantierbare medizinische Vorrichtung, umfassend:
eine oder mehrere Elektroden (22, 24, 42), umfassend eine Gewebe durchdringende Elektrode (12), die von dem Dreieck der Koch-Region des rechten Atriums durch das rechte atriale Endokard und den zentralen Faserkörper implantierbar ist, um an den linken Ventrikel in der basalen Region, septalen Region oder basal-septalen Region des linken ventrikulären Myokards eines Herzens eines Patienten kardiale Therapie abzugeben oder die elektrische Aktivität davon abzufühlen, und
ein abbildbares Element (20), umfassend ein abbildbares Material, das an ein Gehäuse oder eine Zuleitung gekoppelt ist und ausgestaltet ist, um bei Betrachtung unter Verwendung von zweidimensionaler Bildgebung Orientierungsinformationen in einer dritten Dimension bereitzustellen, die orthogonal zu den zwei Dimensionen ist;
eine Therapieabgabeschaltung (84), die funktionell an die eine oder mehreren Elektroden gekoppelt ist, um kardiale Therapie an das Herz des Patienten abzugeben;
eine Abfühlschaltung (86), die funktionell an die eine oder mehreren Elektroden gekoppelt ist, um elektrische Aktivität des Herzens des Patienten abzufühlen; und
eine Steuerung (80), umfassend Verarbeitungsschaltkreis(e), die funktionell an die Therapieabgabeschaltung und die Abfühlschaltung gekoppelt ist/sind, wobei die Steuerung ausgestaltet ist zum:
Abfühlen von elektrischer Aktivität unter Verwendung der einen oder mehreren Elektroden; und
Abgabe von kardialer Therapie basierend auf der abgefühlten elektrischen Aktivität.

2. Vorrichtung nach Anspruch 1, wobei das abbildbare Material röntgendichte Eigenschaften umfasst und unter Verwendung von Fluoroskopie sichtbar ist.

3. Vorrichtung nach einem der vorhergehenden Ansprüche, wobei die eine oder mehreren Elektroden eine rechte atriale Elektrode umfasst/umfassen, die innerhalb des rechten Atriums positionierbar ist, um kardiale Therapie an das rechte Atrium des Herzens des Patienten abzugeben, oder elektrische Aktivität davon abzufühlen.

4. Vorrichtung nach einem der vorhergehenden Ansprüche, wobei die Gewebe durchdringende Elektrode ausgestaltet ist, um bei Betrachtung unter Verwendung von zweidimensionaler Bildgebung Orientierungsinformationen in einer dritten Dimension orthogonal zu den zwei Dimensionen der zweidimensionalen Bildgebung bereitzustellen.

5. Vorrichtung nach einem der vorhergehenden Ansprüche, wobei die Gewebe durchdringende Elektrode eine Spiralform hat.

6. Vorrichtung nach einem der vorhergehenden Ansprüche, des Weiteren umfassend ein Gehäuse (420), wobei die Therapieabgabeschaltung, die Abfühlschaltung und die Steuerung sich innerhalb des Gehäuses befinden und die Gewebe durchdringende Elektrode zuleitungsfrei an das Gehäuse gekoppelt ist.

7. Vorrichtung nach einem der Ansprüche 1 bis 5, des Weiteren umfassend:
ein Gehäuse (420), wobei die Therapieabgabeschaltung, die Abfühlschaltung und die Steuerung sich innerhalb des Gehäuses befinden; und
eine Zuleitung (410), die an das Gehäuse gekoppelt ist und sich von diesem zu einer distalen Endregion erstreckt, wobei die Gewebe durchdringende Elektrode und eine rechte atriale Elektrode an die distale Endregion der Zuleitung gekoppelt sind.

8. Vorrichtung nach einem der Ansprüche 1 bis 5, des Weiteren umfassend:
ein Gehäuse (420), wobei die Therapieabgabeschaltung, die Abfühlschaltung und die Steuerung sich innerhalb des Gehäuses befinden;
eine erste Zuleitung (410), die an das Gehäuse gekoppelt ist und sich von diesem zu einer ersten distalen Endregion erstreckt, wobei die Gewebe durchdringende Elektrode an die erste distale Endregion der ersten Zuleitung gekoppelt ist; und
eine zweite Zuleitung (412), die an das Gehäuse gekoppelt ist und sich von diesem zu einer zweiten distalen Endregion erstreckt, wobei eine rechte atriale Elektrode an die zweite distale Endregion der zweiten Zuleitung gekoppelt ist.

9. Vorrichtung nach einem der Ansprüche 1 bis 5, des Weiteren umfassend:
ein Gehäuse (420), wobei die Therapieabgabeschaltung, die Abfühlschaltung und die Steuerung sich innerhalb des Gehäuses befinden;
eine erste Zuleitung (410), die an das Gehäuse gekoppelt ist und sich von diesem zu einer distalen Endregion erstreckt, wobei die Gewebe durchdringende Elektrode an die erste distale Endregion der ersten Zuleitung gekoppelt ist;
eine zweite Zuleitung (412), die an das Gehäuse gekoppelt ist und sich von diesem zu einer zweiten distalen Endregion erstreckt, wobei eine rechte atriale Elektrode an die zweite distale Endregion der zweiten Zuleitung gekoppelt ist; und
eine dritte Zuleitung (414), die an das Gehäuse gekoppelt ist und sich von diesem zu einer dritten distalen Endregion erstreckt, wobei die eine oder mehreren Elektroden des Weiteren eine rechte ventrikuläre Elektrode umfasst/umfassen, um an den rechten Ventrikel des Herzens des Patienten kardiale Therapie abzugeben oder elektrische Aktivität davon abzufühlen, wobei die rechte ventrikuläre Elektrode an die distale Endregion der dritten Zuleitung gekoppelt ist.

10. Vorrichtung nach einem der Ansprüche 1 bis 9, wobei das abbildbare Element Teil der Gewebe durchdringenden Elektrode ist oder diese einschließt.

## Revendications

1. Dispositif médical implantable comprenant :
une ou plusieurs électrodes (22, 24, 42) comprenant une électrode transperçant des tissus (12) implantable depuis le triangle de la région de Koch de l'atrium droit via l'endocarde atrial droit et un corps fibreux central pour délivrer une thérapie cardiaque à ou pour détecter une activité électrique du ventricule gauche dans la région basale, la région septale ou la région basale-septale du myocarde ventriculaire gauche d'un coeur d'un patient, et un élément pouvant être imagé (20) comprenant un matériel pouvant être imagé couplé à un boîtier ou à un fil configuré, lorsqu'il est visualisé en utilisant une imagerie en deux dimensions, pour fournir des informations d'orientation dans une troisième dimension orthogonale aux deux dimensions ;
un circuit de fourniture de thérapie (84) couplé de manière fonctionnelle aux une ou plusieurs électrodes pour délivrer une thérapie cardiaque au coeur du patient ; un circuit de détection (86) couplé de manière fonctionnelle aux une ou plusieurs électrodes pour détecter une activité électrique du coeur du patient ; et
un contrôleur (80) comprenant une circuiterie de traitement couplée de manière fonctionnelle au circuit de fourniture de thérapie et au circuit de détection, le contrôleur étant configuré pour :
détecter une activité électrique en utilisant les une ou plusieurs électrodes ; et
délivrer une thérapie cardiaque sur la base de l'activité électrique détectée.

2. Dispositif selon la revendication 1, le matériau pouvant être imagé comprenant des propriétés radioopaques visibles en utilisant la fluoroscopie.

3. Dispositif selon l'une quelconque des revendications précédentes, les une ou plusieurs électrodes comprenant une électrode atriale droite pouvant être positionnée dans l'atrium droit pour délivrer une thérapie cardiaque à ou pour détecter une activité électrique de l'atrium droit du coeur du patient.

4. Dispositif selon l'une quelconque des revendications précédentes, l'électrode transperçant des tissus étant configurée, lorsqu'elle est visualisée en utilisant une imagerie en deux dimensions, pour fournir des informations d'orientation dans une troisième dimension orthogonale aux deux dimensions de l'imagerie en deux dimensions.

5. Dispositif selon l'une quelconque des revendications précédentes, l'électrode transperçant des tissus ayant une forme hélicoïdale.

6. Dispositif selon l'une quelconque des revendications précédentes, comprenant en outre un boîtier (420), le circuit de fourniture de thérapie, le circuit de détection et le contrôleur étant situés dans le boîtier et l'électrode transperçant des tissus étant couplée sans fil au boîtier.

7. Dispositif selon l'une quelconque des revendications 1 à 5, comprenant en outre :
un boîtier (420), le circuit de fourniture de thérapie, le circuit de détection et le contrôleur étant situés dans le boîtier ; et
un fil (410) couplé à et s'étendant depuis le boîtier jusqu'à une région d'extrémité distale, l'électrode transperçant des tissus et une électrode atriale droite étant couplées à la région d'extrémité distale du fil.

8. Dispositif selon l'une quelconque des revendications 1 à 5, comprenant en outre :
un boîtier (420), le circuit de fourniture de thérapie, le circuit de détection et le contrôleur étant situés dans le boîtier ;
un premier fil (410) couplé à et s'étendant depuis le boîtier jusqu'à une première région d'extrémité distale, l'électrode transperçant des tissus étant couplée à la première région d'extrémité distale du premier fil ; et
un deuxième fil (412) couplé à et s'étendant depuis le boîtier jusqu'à une deuxième région d'extrémité distale, une électrode atriale droite étant couplée à la deuxième région d'extrémité distale du deuxième fil.

9. Dispositif selon l'une quelconque des revendications 1 à 5, comprenant en outre :
un boîtier (420), le circuit de fourniture de thérapie, le circuit de détection et le contrôleur étant situés dans le boîtier ;
un premier fil (410) couplé à et s'étendant depuis le boîtier jusqu'à une première région d'extrémité distale, l'électrode transperçant des tissus étant couplée à la première région d'extrémité distale du premier fil ;
un deuxième fil (412) couplé à et s'étendant depuis le boîtier jusqu'à une deuxième région d'extrémité distale, l'électrode atriale droite étant couplée à la deuxième région d'extrémité distale du deuxième fil ; et
un troisième fil (414) couplé à et s'étendant depuis le boîtier jusqu'à une troisième région d'extrémité distale, les une ou plusieurs électrodes comprenant en outre une électrode ventriculaire droite pour délivrer une thérapie cardiaque à ou détecter une activité électrique du ventricule droit du coeur du patient couplé à la troisième région d'extrémité distale du troisième fil.

10. Dispositif selon l'une quelconque des revendications 1 à 9, l'élément pouvant être imagé faisant partie de ou comportant l'électrode transperçant les tissus.
